# EUROPEAN PATENT APPLICATION

(11) **EP 3 453 741 A1**
(43) Date of publication of application: **13.03.2019**
(21) Application number: 18769273.6
(22) Date of filing: 29.01.2018
(51) Int. Cl.: C08L 79/08, B29B 17/04, C08J 11/28, C08K 5/05, C08K 5/10, C08K 5/17

(54) **AQUEOUS-SOLVENT SOLUTION OF IMIDE-GROUP-CONTAINING COMPOUND AND PRODUCTION METHOD FOR AQUEOUS-SOLVENT SOLUTION OF IMIDE-GROUP-CONTAINING COMPOUND**

(30) Priority: 12.07.2017 JP 2017136055
(71) Applicant: Nakata Coating Co., Ltd., Yokohama-shi, Kanagawa 240-0041 (JP)
(72) Inventor: MATSUNO Takemi, Yokohama-shi Kanagawa 240-0041 (JP); KITAGAWA Katsuji, Yokohama-shi Kanagawa 240-0041 (JP); HANAZONO Misao, Yokohama-shi Kanagawa 240-0041 (JP)
(74) Representative: Patentanwälte Bressel und Partner mbB
(86) International application number: PCT/JP2018/002652
(87) International publication number: WO 2019/012722

(57) **Abstract**

An aqueous solvent solution of an imide group-containing compound which contains an imide group-containing compound produced by partial hydrolysis of a polyimide molded article is provided.

Provided are an aqueous solvent solution of an imide group-containing compound and a method for producing the same including steps of: (1) preparing a polyimide molded article by cutting the polyimide molded article into a predetermined size, (2) hydrolyzing the polyimide molded article at temperature conditions of 50 to 100°C in the presence of water and a basic compound to have a crude imide group-containing compound in solution state, (3) purifying the crude imide group-containing compound in solution state to have an imide group-containing compound in semi-solid state, and (4) dissolving the imide group-containing compound in semi-solid state, an aqueous solvent, and a predetermined alkali compound to have a predetermined aqueous solvent solution.

## Description

### TECHNICAL FIELD

The present invention relates to an aqueous solvent solution of a compound containing an imide group (or an imide group-containing compound, hereinbelow, it may be referred to as an aqueous solution of an imide group-containing compound, or simply referred to as a predetermined aqueous solvent solution), and a method for producing an aqueous solvent solution of a compound containing an imide group(or an imide group-containing compound).

In particular, the invention relates to an aqueous solvent solution of an imide group-containing compound which is obtained by containing a specific imide group-containing compound that can be cured at low temperature and has excellent solubility in water (also simply referred to as an aqueous solvent solution or an aqueous solution, and ditto for the followings), and a method for efficient production thereof.

### BACKGROUND ART

Hitherto, polyimide molded articles represented by polyimide films are insoluble not only in an aqueous solvents but also in various kinds of organic solvents due to the excellent chemical resistance thereof, and, unlike thermoplastic plastics such as polystyrene, polyimide molded articles are hardly recycled by melting due to a high melting point thereof.

For this reason, the polyimide molded articles are mostly discarded by landfill disposal or incineration despite a high price and thus have a problem of being poor in recyclability or environmental properties.

In view of such a circumstance, various methods have been proposed in which the polyimide molded articles to be discarded are recycled by the chemical hydrolysis.

For example, to provide polyimide powder of which average particle diameter is equal to or less than a predetermined value, as an aggregate of fine particles produced by depositing polyimide dissolved in a treatment liquid containing a basic substance, polyimide powder of which fine particles contain polyimide and polyamide acid and residual amount of an alkali metal of a basic substance contained in the treatment liquid is equal to or less than 1% relative to the whole powder amount, and a method for producing the polyimide powder are suggested (see, Patent Document 1).

More specifically, the polyimide powder has particle size distribution of 1 to 500 µm and average particle diameter (median diameter: D₅₀) of 25 µm or less, for example.

Furthermore, to enhance the storage stability of an aqueous dispersion composition, an aqueous dispersion composition obtained by blending an amine (B) in a specific polyimide resin (A) is suggested (see, Patent Document 2).

More specifically, to enhance the storage stability of an aqueous dispersion composition, an aqueous dispersion composition containing a polyimide resin (A), which has an alkylene glycol bisanhydrotrimellitate residue and a diisocyanate residue and an acid number of 15 to 100 mgKOH/g, and an amine (B) in an amount of 1 to 10 equivalents relative to the acid number of the polyimide resin (A) is suggested.

Furthermore, to enhance the storage stability of a polyimide precursor varnish, as a polyimide precursor consisting of a reaction product with an organic solvent, a polyimide precursor varnish obtained by containing water, an amine compound, and an organic solvent is suggested (see, Patent Document 3).

More specifically, a polyimide precursor varnish consisting of 99 to 50% by weight of a polyimide precursor, which consists of polyamide acid, 0.6 to 1.0 equivalent of an amine compound relative to carboxyl group of the polyamide acid, and 0.2 to 1.0 equivalent of an organic solvent relative to amide group of the polyamide acid, and 1 to 50% by weight of water as a solvent is suggested.

### CITATION LIST

### PATENT DOCUMENT

Patent Document 1: JP 5695675 B2 (claims)
Patent Document 2: JP 2016-199749 A (claims)
Patent Document 3: JP 8-291252 A (claims)

### DISCLOSURE OF THE INVENTION

### PROBLEM TO BE SOLVED BY THE INVENTION

However, with respect to the polyimide powder and a method for producing the same that are described in Patent Document 1, as an aggregate of fine particles containing polyimide and polyamide acid produced by depositing polyimide dissolved in a treatment liquid containing a basic substance followed by drying the polyimide, it is necessary to produce polyimide powder of which average particle diameter is equal to or less than 25 µm.

As such, for production of a predetermined polyimide solution, there have been problems that, after producing the polyimide with such particle shape, it has to be dissolved in a solvent, and excessively large number of production steps is required and energy loss is excessively high.

In addition, the polyimide powder disclosed in Patent Document 1 has insufficient reactivity, and similar to common polyimide, it needs to heated for 60 minutes or longer at a temperature of 200°C to have the reaction progressed at certain level.

Meanwhile, with regard to the aqueous dispersion composition of Patent Document 2, it is necessary to use a very specific polyimide compound, and, to enhance storage stability of the compound, it is disclosed that a predetermined amine compound is blended.

However, with regard to the enhancement of water solubility of a predetermined imide group-containing compound in an aqueous solvent or the effect of increasing the curing speed of an imide group-containing compound (for example, low temperature curability), and also the enhancement of film forming property, neither description nor suggestion is included therein.

Furthermore, it is also described that a predetermined amine compound is blended with the polyimide precursor of Patent Document 3, in order to enhance the storage stability of the polyimide precursor.

However, also in Patent Document 3, neither description nor suggestion is included with regard to the enhancement of water solubility of a predetermined imide group-containing compound in an aqueous solvent or the effect of increasing the curing speed of an imide group-containing compound (for example, low temperature curability), and also the enhancement of film forming property,.

Furthermore, from the viewpoint that a significant amount of an organic solvent needs to be included, a problem may occur in terms of safety, or there is a problem like limited use due to the likelihood of being left as residuals.

In this regard, as a result of intensive studies, the inventors of the invention found that, by preparing a predetermined aqueous solvent solution according to blending of a predetermined imide group-containing compound, an amine compound having predetermined boiling point, and an aqueous solvent, low temperature curability is obtained, and favorable solubility in an aqueous solvent or storage solubility is obtained at the same time, and also a favorable film forming property is obtained. The invention is completed accordingly.

Namely, an object of the invention is, in order to have low temperature curability and excellent solubility in water and also to obtain a favorable film forming property, to provide an aqueous solvent solution of an imide group-containing compound obtained by containing a specific imide group-containing compound, an amine compound having predetermined boiling point, and an aqueous solvent, and a method for efficient production of a predetermined aqueous solvent solution.

### MEANS FOR SOLVING PROBLEM

According to the invention of the present application, provided is an aqueous solvent solution of an imide group-containing compound which contains an imide group-containing compound obtained by partial hydrolysis of a polyimide molded article, an amine compound of which boiling point (at normal pressure, and ditto for the followings) is a value within the range of from 85 to 145°C, and an aqueous solvent, characterized in that the imide group-containing compound has, in an infrared spectroscopy chart, an absorption peak at a wave number of 1,500 cm⁻¹ which is derived from a benzene ring and an absorption peak at a wave number of 1,375 cm⁻¹ which is derived from an imide group, and, at the same time, when the height of the absorption peak at a wave number of 1,500 cm⁻¹ which is derived from a benzene ring is denoted as S1 and the height of the absorption peak at a wave number of 1,375 cm⁻¹ which is derived from an imide group is denoted as S2, the ratio of S1/S2 is a value within the range of from 2 to 10.

By having a constitution that an amine compound with a boiling point similar to the boiling point of water (100°C at normal pressure) is contained, an imide group-containing compound reacts with the amine compound to form an amine salt, and, at the same time, the evaporation property of water/amine compound can be controlled. Furthermore, it is possible to have a predetermined aqueous solvent solution which has a favorable low temperature curability (imidation rate: 70% or higher), a favorable solubility in water, and also a favorable film forming property or the like.

Namely, by having a predetermined aqueous solvent solution which is obtained by partial hydrolysis of a polyimide molded article of an industrial waste or the like and also by containing an imide group-containing compound having a predetermined infrared absorption peak, an aqueous solvent, and an amine compound having a predetermined boiling point, when a film is formed, it can contribute, as an amine salt of an imide group-containing compound, to the low temperature curability without having excessive scattering or remaining of an amine compound.

Furthermore, for the same reason as above, as the amine compound suitably remains even in a case in which a film is formed, it is possible to have a predetermined aqueous solvent solution which maintains favorable solubility in water and also allows obtainment of a favorable film forming property.

It is also found that, with an imide group-containing compound derived from the predetermined aqueous solvent solution, a predetermined polyimide resin can be obtained by thermal curing at a temperature of 200°C or lower, but, a polyimide resin exhibiting heat resistance or the like equivalent to those of a related art is obtained at a cost which is 1/10 or less of the cost for producing the polyimide resin of a related art.

As such, it can be said that, as a raw material of a recycled polyimide resin or a raw material of a polyimide resin which exhibits novel characteristics, the aqueous solvent solution of an imide group-containing compound is very advantageous from the economic point of view.

Furthermore, the partial hydrolysis of a polyimide molded article can be identified by confirming the presence of the aforementioned absorption peak derived from an imide group, absorption peak derived from an amide group, and absorption peak derived from a carboxy group in an infrared spectroscopy chart.

For constituting the aqueous solvent solution of an imide group-containing compound of the invention of the present application, the aqueous solvent is preferably water and an alcohol compound, or either one of them.

By having this constitution, the imide group-containing compound can be homogeneously dissolved, and, at the same time, a predetermined aqueous solvent solution which has high safety and high economic value can be provided.

The amine compound is preferably N,N-dimethylaminoethanol (boiling point: 133°C) and trimethylamine (boiling point: 89.5°C), or either one of them.

By having this constitution, the predetermined aqueous solvent solution can be cured at low temperature, can have more favorable solubility in water, and can have a favorable film forming property.

There is also an advantage that those amine compounds have only a slight malodor, good handlability, and safety.

For constituting the aqueous solvent solution of an imide group-containing compound of the invention of the present application, it is preferable that, in an infrared spectroscopy chart of the imide group-containing compound, there is an absorption peak at a wave number of 1,600 cm⁻¹ which is derived from an amide group, and, at the same time, when the height of the absorption peak at a wave number of 1,500 cm⁻¹ which is derived from a benzene ring is denoted as S1 and the height of the absorption peak at a wave number of 1,600 cm⁻¹ which is derived from an amide group is denoted as S3, the ratio of S1/S3 is a value within the range of from 2 to 20.

By having this constitution, an imide group-containing compound which is derived from an aqueous solvent solution of an imide group-containing compound and can be cured at even lower temperature can be obtained, and, at the same time, it can be employed as an indicator showing the presence or absence of partial hydrolysis of a polyimide molded article.

Furthermore, depending on the type of a polyimide constituting a polyimide molded article, that is, type of the imide group-containing compound obtained by partial hydrolysis, the absorption peak at a wave number of 1,500 cm⁻¹ which is derived from a benzene ring (standard peak) may not appear. Thus, for such case, another absorption peak may be taken as a standard peak instead of the absorption peak at a wave number of 1,500 cm⁻¹ which is derived from a benzene ring.

For constituting the aqueous solvent solution of an imide group-containing compound of the invention of the present application, it is preferable that, in an infrared spectroscopy chart of the imide group-containing compound, there is an absorption peak at a wave number of 1,413 cm⁻¹ which is derived from a carboxy group, and, at the same time, when the height of the absorption peak at a wave number of 1,500 cm⁻¹ which is derived from a benzene ring is denoted as S1 and the height of the absorption peak at a wave number of 1,413 cm⁻¹ which is derived from a carboxyl group is denoted as S4, the ratio of S1/S4 is a value within the range of from 8 to 30.

By having this constitution, an imide group-containing compound which is derived from an aqueous solvent solution of an imide group-containing compound and has more excellent solubility in a predetermined organic solvent or more excellent adhesiveness to various substrates can be obtained, and, at the same time, it can be employed as an indicator showing the presence or absence of partial hydrolysis of a polyimide molded article.

For constituting the aqueous solvent solution of an imide group-containing compound of the invention of the present application, the blending amount of the aqueous solvent is preferably set at a value that is within the range of from 20 to 99% by weight relative to the whole amount.

By having this constitution, viscosity of a predetermined aqueous solvent solution can be adjusted to a value within a predetermined range, easy handlability is obtained, or a uniform coating film can be formed.

For constituting the aqueous solvent solution of an imide group-containing compound of the invention of the present application, the blending amount of the amine compound is preferably set at a value that is within the range of from 0.1 to 25% by weight relative to the whole amount.

By having this constitution, viscosity of a predetermined aqueous solvent solution can be adjusted to a value within a predetermined range, easy handlability is obtained, or a uniform coating film can be formed.

Another aspect of the invention of the present application is a method for producing an aqueous solvent solution of an imide group-containing compound, including the followings steps (1) to (3):
(1) a step of preparing a polyimide molded article by cutting the polyimide molded article into a predetermined size,
(2) a step of hydrolyzing the polyimide molded article at temperature conditions of 50 to 100°C in the presence of water and a basic compound to have an imide group-containing compound in solution state, and
(3) a step of blending the imide group-containing compound with an amine compound of which boiling point is a value within the range of from 85 to 145°C and an aqueous solvent to obtain an aqueous solvent solution of an imide group-containing compound,
characterized in that the imide group-containing compound obtained from the step (2) has, in an infrared spectroscopy chart, an absorption peak at a wave number of 1,500 cm⁻¹ which is derived from a benzene ring and an absorption peak at a wave number of 1,375 cm⁻¹ which is derived from an imide group, and, at the same time, when the height of the absorption peak at a wave number of 1,500 cm⁻¹ which is derived from a benzene ring is denoted as S1 and the height of the absorption peak at a wave number of 1,375 cm⁻¹ which is derived from an imide group is denoted as S2, the ratio of S1/S2 is a value within the range of from 2 to 10.

Namely, by producing an aqueous solvent solution of an imide group-containing compound as described above, the imide group-containing compound and amine compound form an amine salt, and, at the same time, the evaporation property of water/amine compound can be controlled and a favorable low temperature curability can be obtained, and also a predetermined aqueous solvent solution which has favorable solubility in water or a favorable film forming property can be efficiently produced.

Furthermore, with an imide group-containing compound derived from a predetermined aqueous solvent solution, thermal curing can be achieved even at a low temperature condition like 200°C or lower, but accordingly, heat resistance, mechanical properties or ultraviolet absorption property, or the like that are equivalent to those of a common polyimide resin can be obtained.

As such, it is found that a polyimide resin having heat resistance or the like equivalent to those of a related art is obtained at a cost which is 1/10 or less of the cost for producing the polyimide resin of a related art, and thus it can be said that the above method is very advantageous also from the economic point of view.

### BRIEF DESCRIPTION OF DRAWINGS

Fig. 1 is a diagram illustrating the relation between the blending amount of an amine compound of which boiling point is a value within a predetermined range (N,N-dimethylaminoethanol) in an aqueous solvent solution of an imide group-containing compound and the viscosity (measurement temperature: 25°C) of the aqueous solvent solution of an imide group-containing compound;
Fig. 2 is a diagram illustrating the relation between the blending amount of an amine compound of which boiling point is a value within a predetermined range (N,N-dimethylaminoethanol) in an aqueous solvent solution of an imide group-containing compound and the low temperature curability of the imide group-containing compound;
Fig. 3 is an infrared spectroscopy chart of an imide group-containing compound (compound A) according to the invention (Example 1);
Fig. 4 is an infrared spectroscopy chart of the cured product (polyimide resin) of an imide group-containing compound (compound A) according to the invention (Example 1);
Fig. 5 is a diagram provided to describe the relation between the time elapsed (days) and the viscosity (mPa·sec) of the imide group-containing compound solution during storage;
Fig. 6 is a diagram provided to describe the relation between the blending amount of a viscosity stabilizer (trimethyl orthoformate) in the imide group-containing compound solution and the viscosity thereof;
Fig. 7 is a diagram provided to describe the relation between the blending amount of a viscosity stabilizer (triethyl orthoformate) in the imide group-containing compound solution and the viscosity thereof;
Figs. 8(a) to 8(e) are diagrams provided to describe the aspects of the use of an imide group-containing compound;
Fig. 9 is an infrared spectroscopy chart of a commercially available polyimide (KAPTON H);
Fig. 10 is a differential scanning thermogravimetry chart (TG-DTA curve) of the cured product (polyimide resin) of an imide group-containing compound (compound A) according to the invention (Example 1);
Fig. 11 is an infrared spectroscopy chart of an imide group-containing compound (compound B) according to the invention (Example 2);
Fig. 12 is an infrared spectroscopy chart of the thermally cured product (polyimide resin) of an imide group-containing compound (compound B) according to the invention (Example 2);
Fig. 13 is an infrared spectroscopy chart of an imide group-containing compound (compound C) according to the invention (Example 3);
Fig. 14 is an infrared spectroscopy chart of the thermally cured product (polyimide resin) of an imide group-containing compound (compound C) according to the invention (Example 3); and
Fig. 15 is an infrared spectroscopy chart of an imide group-containing compound (compound D) of Comparative Example 2.

### MODE(S) FOR CARRYING OUT THE INVENTION

### [First embodiment]

The first embodiment is an aqueous solvent solution of an imide group-containing compound which contains an imide group-containing compound obtained by partial hydrolysis of a polyimide molded article, an aqueous solvent, and an amine compound of which boiling point is a value within the range of from 85 to 145°C, characterized in that the imide group-containing compound has, in an infrared spectroscopy chart, an absorption peak at a wave number of 1,500 cm⁻¹ which is derived from a benzene ring and an absorption peak at a wave number of 1,375 cm⁻¹ which is derived from an imide group, and, at the same time, when the height of the absorption peak at a wave number of 1,500 cm⁻¹ which is derived from a benzene ring is denoted as S1 and the height of the absorption peak at a wave number of 1,375 cm⁻¹ which is derived from an imide group is denoted as S2, the ratio of S1/S2 is a value within the range of from 2 to 10.

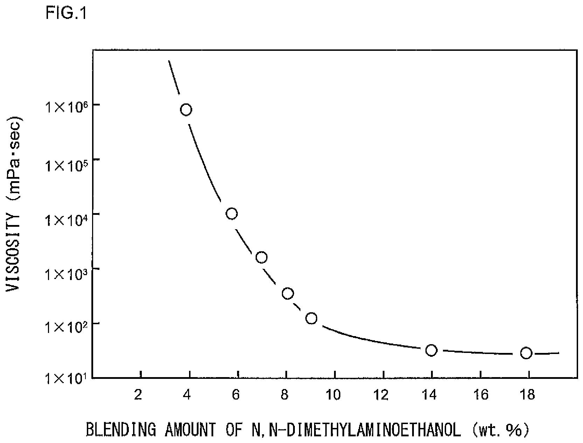

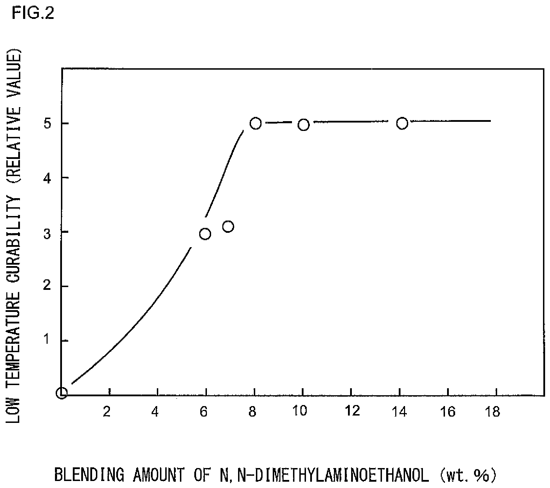

Namely, as illustrated in Fig. 1 and Fig. 2, by containing an amine compound which has a boiling point at predetermined temperature, viscosity of the aqueous solvent solution of an imide group-containing compound can be adjusted to a desired range, and also, favorable low temperature curability, solubility in an aqueous solvent, and film forming property, or the like can be obtained, respectively.

### 1. Polyimide molded article

For producing an imide group-containing compound by partial hydrolysis, a polyimide molded article which has been conventionally treated as an industrial waste is widely employed as a raw material.

Hence, examples of the suitable polyimide molded article may include a polyimide film, a polyimide coating, a polyimide-based resist, a polyimide electrical part enclosure, a polyimide electronic part material, a polyimide container, a polyimide machine part, and a polyimide automobile part.

Moreover, a composite laminate such as a circuit board having a metal circuit pattern formed on a surface of the polyimide film surface or a TAB tape can also be used as a polyimide molded article as the raw material at the time of the production of the imide group-containing compound of the invention.

### 2. Partial hydrolysis

### (1) Predetermined structure

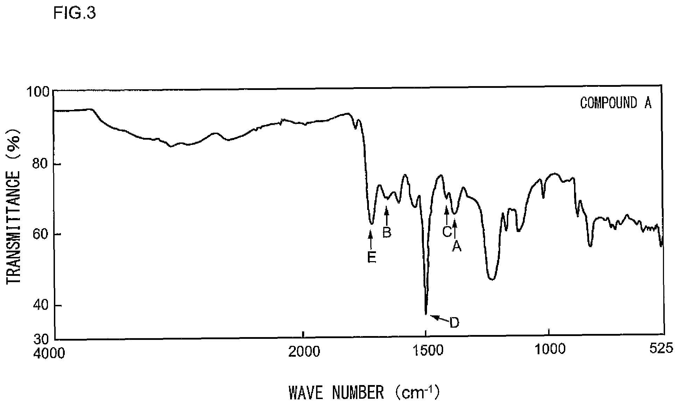

In addition, the imide group-containing compound of the invention is a partial hydrolyzate of a polyimide molded article having a predetermined structure as the infrared spectroscopy chart is illustrated in Fig. 3.

Namely, it is an imide group-containing compound obtained by partially hydrolyzing a polyimide molded article having a predetermined size in the presence of water and a basic compound under a temperature condition of from 50 to 100°C even though it is an example, and the imide group-containing compound which has a predetermined structure and is represented by the following formula (1) is the subject.

As such, by having at least an imide group, an amide group, and a carboxyl group, and further a carbonyl group or the like in the molecule composed of a carbon atom or the like, it is possible to obtain an imide group-containing compound which is curable at low temperature and exhibits excellent solubility in various kinds of aqueous solvents or adhesiveness to various adherends. 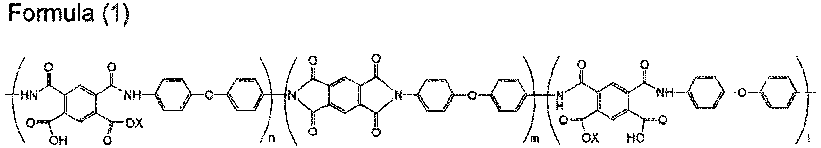 (in the formula (1), the symbol X represents an alkali metal (Lithium/Li, sodium/Na, potassium/K, rubidium/Rb, or cesium/Ce) or the like, the subscript n and 1 represent a symbol that denotes the abundance (number of moles) of the polyamide acid structure located on the both sides of the polyimide structure and are usually a value within the range of from 0.1 to 0.8, and the subscript m represents a symbol that indicates the abundance (number of moles) of the polyimide structure and is usually a value within the range of from 0.2 to 0.9).

In addition, it is believed that the imide group-containing compound has a predetermined structure at the molecular terminal as represented by the following formula (2) .

Namely, it is believed that the molecular terminal structure is constituted with one or a combination of a polyamide acid structure denoted by the symbol A, a mixture of a polyamide acid and an alkali soap structure denoted by the symbol B, and an alkali soap structure denoted by the symbol C.

As such, by having such a molecular terminal, it is possible to obtain an imide group-containing compound which is curable at further lower temperature and exhibits more excellent solubility in various kinds of aqueous solvents or adhesiveness to various adherends.

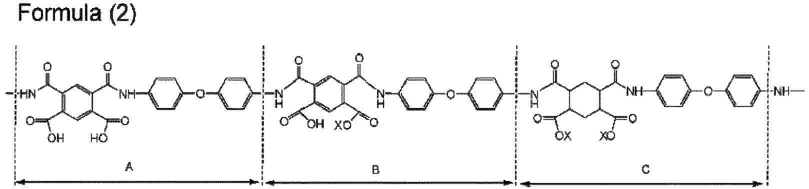

However, the imide group-containing compound does not necessarily contain at the same time the imide group, the amide group, and the carboxyl group in one molecule composed of a carbon atom but may be a mixture of a polyimide having an imide group represented by the following formula (3)-1, a polyamide acid having an amide group represented by the following formula (3)-2, and a carboxylic acid compound having a carboxyl group represented by the following formula (3)-3.

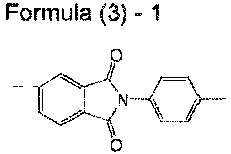

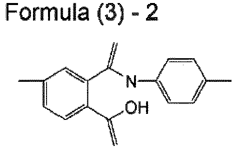

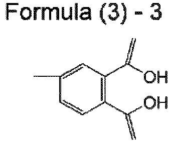

### (2) Infrared spectroscopy chart

### (2)-1 Imide group

In addition, the imide group-containing compound of the invention is characterized in that it has an absorption peak derived from an imide group at a wave number of 1,375 cm⁻¹ or in the vicinity thereof in the infrared spectroscopy chart obtained by the infrared spectroscopic measurement as illustrated in Fig. 3.

The reason for this is because it is possible to obtain an imide group-containing compound which is lower-temperature curable and thus to exhibit a predetermined heat resistance in a case in which a polyimide resin having a high molecular weight is obtained by the thermal curing treatment by having an imide group in the molecule as described above.

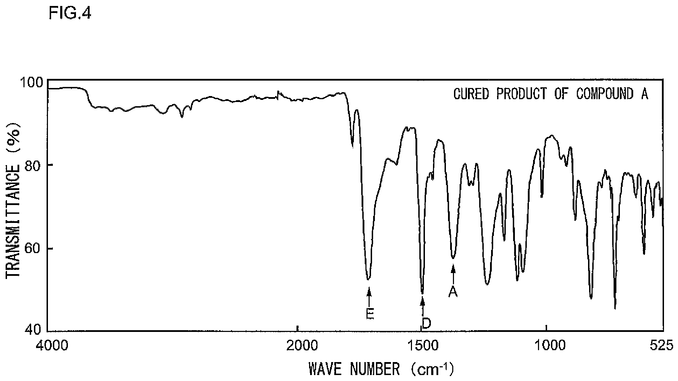

Furthermore, it is also possible to use the amount (peak height) of the imide group in the imide group-containing compound of the invention as an indicator showing the degree of partial hydrolysis as illustrated in the infrared spectroscopy chart of Fig. 3. However, when the quantity (peak height) of the imide group in the infrared spectroscopy chart of the polyimide after curing illustrated in Fig. 4 or the like is 100, it has been found that the amount is preferably within the range of from 10 to 50, more preferably within the range of from 15 to 45, and even more preferably within the range of from 20 to 40.

### (2)-2 Amide group

In addition, the imide group-containing compound of the invention is characterized in that it has an absorption peak derived from an amide group at a wave number of 1,600 cm⁻¹ or in the vicinity thereof as illustrated in Fig. 3.

The reason for that is because it is possible to obtain an imide group-containing compound which is curable at lower temperature by having an amide group in the molecule as described above.

Meanwhile, it has been found that an absorption peak attributed to the amide group is not included in the polyimide obtained by curing as illustrated in Fig. 4 although a distinctive absorption peak (wave number of 1,600 cm⁻¹) attributed to the amide group is denoted as shown from the imide group-containing compound of the invention as illustrated in the infrared spectroscopy chart of Fig. 3.

### (2)-3 Carboxyl group

In addition, the imide group-containing compound of the invention is characterized in that it has an absorption peak derived from a carboxyl group at a wave number of 1,413 cm⁻¹ or in the vicinity thereof as illustrated in Fig. 3.

The reason for that is because it is possible to obtain an imide group-containing compound exhibiting favorable solubility or adhesiveness by having a carboxyl group in the molecule as described above.

Furthermore, it has been found that an absorption peak attributed to the carboxyl group is not included in the polyimide obtained by curing as illustrated in Fig. 4 although an absorption peak (wave number of 1,413 cm⁻¹) attributed to the carboxyl group is denoted as shown from the imide group-containing compound of the invention as illustrated in the infrared spectroscopy chart of Fig. 3.

### (2)-4 Carbonyl group

In addition, it is preferable that the imide group-containing compound of the invention has an absorption peak derived from a carbonyl group at a wave number of 1,710 cm⁻¹ or in the vicinity thereof as illustrated in Fig. 3.

The reason for that is because it is possible to obtain an imide group-containing compound exhibiting more favorable solubility by having a carbonyl group in the molecule as described above.

Furthermore, it is also possible to use the quantity (peak height) of the carbonyl group in the imide group-containing compound of the invention as an indicator showing the degree of partial hydrolysis as illustrated in the infrared spectroscopy chart of Fig. 3.

Thus, it has been found that the quantity (peak height) of the carbonyl group is preferably within the range of from 30 to 70, more preferably within the range of from 35 to 60, and even more preferably within the range of from 40 to 50 when the quantity (peak height) of the carbonyl group in the infrared spectroscopy chart of the polyimide illustrated in Fig. 3 is 100.

### (2)-5 Ratio to benzene ring

### (S1/S2)

In addition, it is preferable that the ratio of S1/S2 is a value within the range of from 2 to 10 when the height of the absorption peak derived from a benzene ring at the wavenumber of 1,500 cm⁻¹ is denoted as S1 and the height of the absorption peak derived from an imide group at the wavenumber of 1,375 cm⁻¹ is denoted as S2 in the infrared spectroscopy chart of the imide group-containing compound of the invention.

The reason for this is because it is also possible to use the ratio of S1/S2 as an indicator indicating the degree of partial hydrolysis as well as it is possible to obtain an imide group-containing compound which is lower-temperature curable by regulating the abundance proportion of the imide group as described above.

Consequently, the ratio of S1/S2 is more preferably a value within the range of from 3 to 8 and even more preferably a value within the range of from 5 to 7.

### (S1/S3)

In addition, it is preferable that the ratio of S1/S3 is a value within the range of from 2 to 20 when the height of the absorption peak derived from a benzene ring at the wavenumber of 1,500 cm⁻¹ is denoted as S1 and the height of the absorption peak derived from an amide group at the wavenumber of 1,600 cm⁻¹ is denoted as S3 in the infrared spectroscopy chart of the imide group-containing compound of the invention.

The reason for this is because it is also possible to use the ratio of S1/S3 as an indicator indicating the degree of partial hydrolysis as well as it is possible to obtain an imide group-containing compound exhibiting further excellent solubility in a predetermined organic solvent or adhesiveness by regulating the abundance proportion of the amide group as described above.

Consequently, the ratio of S1/S3 is more preferably a value within the range of from 5 to 15 and even more preferably a value within the range of from 7 to 12.

### (S1/S4)

In addition, it is preferable that the ratio of S1/S4 is a value within the range of from 8 to 30 when the height of the absorption peak derived from a benzene ring at the wavenumber of 1,500 cm⁻¹ is denoted as S1 and the height of the absorption peak derived from a carboxyl group at the wavenumber of 1,413 cm⁻¹ is denoted as S4 in the infrared spectroscopy chart of the imide group-containing compound of the invention.

The reason for this is because it is also possible to use the ratio of S1/S4 as an indicator indicating the degree of partial hydrolysis as well as it is possible to obtain an imide group-containing compound exhibiting further excellent solubility in a predetermined organic solvent or adhesiveness by regulating the abundance proportion of the carboxyl group as described above.

Consequently, the ratio of S1/S4 is more preferably a value within the range of from 10 to 25 and even more preferably a value within the range of from 13 to 20.

### (3) Average weight molecular weight

In addition, it is preferable that the average weight molecular weight of the imide group-containing compound of the invention is a value within the range of from 1,000 to 100,000.

The reason for this is because favorable solubility of the imide group-containing compound in an organic solvent is obtained as well as predetermined low-temperature curability thereof is obtained by having an average weight molecular weight within this range.

Consequently, the average weight molecular weight of the imide group-containing compound is more preferably a value within the range of from 3,000 to 60,000 and even more preferably a value within the range of from 5,000 to 30,000.

Meanwhile, the average weight molecular weight of the imide group-containing compound can be measured by gel permeation chromatography in terms of polystyrene.

### 3. Aqueous solvent

### (1) Type

In addition, the type of the aqueous solvent in the aqueous solvent solution of an imide group-containing compound is not particularly limited but is preferably at least one of water and alcohol.

Furthermore, although it is believed to be due to the reason that the amine compound having a predetermined boiling point is blended even when water is singly present as the aqueous solvent, water as a single aqueous solvent is appropriate in that it is a good solvent for the imide group-containing compound and is excellent in terms of the safety or environmental property.

Other than the above, it is also preferable that the imide group-containing compound is dissolved in advance by using a relatively small amount of an organic solvent like N-methyl-2-pyrrolidone or the like (for example, 5% by weight or less, and more preferably 1% by weight or less of the total amount of a predetermined aqueous solvent solution), and water, alcohol or the like is additionally blended in that state so as to have a predetermined amount (for example, 55 to 94% by weight of the total amount), thereby obtaining an aqueous solution or alcohol solution of the imide group-containing compound which is uniform and exhibits high safety or the like.

### (2) Blending amount

In addition, with regard to the blending amount of the aqueous solvent, it is preferable to add the aqueous solvent so as to have a value within the range of from 1 to 40% by weight in terms of the solid content concentration of the imide group-containing compound.

The reason for that is because the effect of the viscosity stabilizer can be effectively exhibited and thus favorable storage stability can be obtained by adjusting the blending amount of the aqueous solvent such that the solid content concentration of the imide group-containing compound is a value within a predetermined range.

In addition, that is because, when the solid content concentration of the imide group-containing compound is within this range, not only handling but also coating and drying of the aqueous solvent solution of an imide group-containing compound are facilitated and further it is possible to uniformly and rapidly blend another blending component, for example, a thermoplastic resin component, a thermocurable resin component, a photocurable resin component, a metal material, and a ceramic material.

More specifically, that is because, when the blending amount of the aqueous solvent is a value of above 99% by weight and the solid content concentration of the imide group-containing compound is a value of below 1% by weight, there is a case in which the handling of the aqueous solvent solution of an imide group-containing compound is difficult due to the excessively low initial viscosity thereof or coating and drying are insufficient at the time of forming the polyimide film.

On the other hand, that is because, when the blending amount of the aqueous solvent is a value of below 60% by weight and the solid content concentration of the imide group-containing compound is a value of above 40% by weight, there is a case in which the effect of the viscosity stabilizer may not be effectively exhibited so that the viscosity stability of the aqueous solvent solution of an imide group-containing compound significantly deteriorates.

As such, with regard to the blending amount of the aqueous solvent, the solvent is blended such that the solid content concentration is more preferably a value within the range of from 5 to 30% by weight and even more preferably a value within the range of from 10 to 20% by weight relative to the total amount of the aqueous solvent solution of an imide group-containing compound in terms of the solid content concentration of the imide group-containing compound.

### 4. Amine compound

### (1) Type

In addition, it is also characterized in that an amine compound having a predetermined boiling point is blended in the aqueous solvent solution of an imide group-containing compound.

That is due to the reason of having an amination of the amine compound having a predetermined boiling point with a function group contained in an imide group-containing compound, and further enhancing the low temperature curability, solubility in an aqueous solvent, film forming property, viscosity stability, or the like.

As such, it is preferable to blend an amine compound which has a boiling point that is similar to the boiling point of water, or a boiling point slightly higher than the boiling point of water (for example, within 45°C, and more preferably within 35°C), and more specifically an amine compound which has a boiling point within the range of from 85 to 145°C.

Namely, at least one of alkylamines or alkanolamines such as butylamine (78°C), triethylamine (boiling point: 89.5°C), N-methylmorpholine (115°C), N,N,N',N'-tetramethylethylenediamine (121°C), N,N-dimethylaminoethanol (boiling point: 133°C), N,N-diethylethanolamine (boiling point: 134°C), or cyclohexanolamine (boiling point: 134.5°C) is preferable.

In particular, N,N-dimethylaminoethanol (boiling point: 133°C) has only a slight malodor and, even in a relatively small amount (for example, 8 to 16% by weight in the whole amount), it can easily yield an amination with a functional group of the imide group-containing compound, and thus the low temperature curability, solubility in an aqueous solvent, film forming property, viscosity stability, or the like can be enhanced more.

In addition, as it has higher boiling point than the boiling point of water (100°C) and, even when a film is formed by coating or the like to have a predetermined thickness at a predetermined temperature (200°C or lower) or impregnated or the like, water is more prone to scattering and N,N-dimethylaminoethanol is also scattered in the end so that it is not likely to be remained, and thus favorable low temperature curability, solubility in an aqueous solvent, or the like can be maintained even during the production steps.

### (2) Blending amount 1

Furthermore, it is also desirable that the blending amount of the amine compound having a predetermined boiling point is determined by considering the whole amount of the aqueous solvent solution of an imide group-containing compound.

As such, it is desirable that the blending amount of the amine compound having a predetermined boiling point is a value within the range of from 0.1 to 25% by weight relative to the whole amount of the aqueous solvent solution of an imide group-containing compound (100% by weight).

That is because, when the blending amount of the amine compound is a value lower than 0.1% by weight, depending on the type or the like of the amine compound, the viscosity stabilizing effect of the amine compound may be significantly reduced so that the aqueous solvent solution of an imide group-containing compound may turn into a gel phase.

On the other hand, when the blending amount of the amine compound is above 25% by weight, there may be a case in which the heat resistance or mechanical property of a cured product derived from the imide group-containing compound is significantly impaired or malodor becomes excessively strong.

As such, the blending amount of the amine compound is more preferably a value within the range of 1 to 18% by weight, even more preferably is a value within the range of 4 to 15% by weight, still even more preferably is a value within the range of 6 to 12% by weight, and most preferably is a value within the range of 7 to 10% by weight relative to the whole amount of the aqueous solvent solution of an imide group-containing compound (100% by weight).

Herein, with reference to Fig. 1, the relation between the blending amount of an amine compound of which boiling point is a value within a predetermined range (N,N-dimethylaminoethanol) in an aqueous solvent solution of an imide group-containing compound and the viscosity (measurement temperature: 25°C) of the aqueous solvent solution of an imide group-containing compound is explained.

Although the basic composition of the aqueous solvent solution of an imide group-containing compound is based on Example 1 or the like, in Fig. 1, the horizontal axis represents the blending amount of a predetermined amine compound and the vertical axis represents the viscosity (25°C) of the aqueous solvent solution of an imide group-containing compound.

In addition, when the blending amount of a predetermined amine compound is 6% by weight or so, the viscosity of the aqueous solvent solution of an imide group-containing compound is approximately 5×10⁵ mPa·sec.

Similarly, when the blending amount of a predetermined amine compound is increased to 7% by weight or so, the viscosity is significantly lowered, showing the viscosity of approximately 2×10⁴ mPa·sec.

Furthermore, when the blending amount of a predetermined amine compound is additionally increased to 8% by weight or so, the viscosity is more significantly lowered, showing the viscosity of approximately 7×10² mPa·sec.

Furthermore, when the blending amount of a predetermined amine compound is additionally increased to 9 to 10% by weight or so, the viscosity is further lowered, but showed the viscosity of approximately 1×10² mPa·sec.

Still furthermore, when the blending amount of a predetermined amine compound is additionally increased to 14% by weight or so, the decrease amount of viscosity is significantly reduced, showing a significantly saturated tendency, and the viscosity is approximately 7×10² mPa·sec.

Accordingly, from the characteristic curve shown in Fig. 1, it is understood that an aqueous solvent solution of an imide group-containing compound with desired viscosity can be obtained by suitably modifying the blending amount of a predetermined amine compound.

Subsequently, with reference to Fig. 2, the relation between the blending amount of an amine compound of which boiling point is a value within a predetermined range (N,N-dimethylaminoethanol) in an aqueous solvent solution of an imide group-containing compound and the low temperature curability of the imide group-containing compound is explained.

Although the basic composition of the aqueous solvent solution of an imide group-containing compound is based on Example 1 or the like, in Fig. 2, the horizontal axis represents the blending amount of a predetermined amine compound and the vertical axis represents the low temperature curability (relative value) of the imide group-containing compound.

The relative value of the low temperature curability was calculated as follows: the evaluation ⊙ is given with 5 points, the evaluation ○ is given with 3 points, the evaluation Δ is given with 1 point, and the evaluation × is given with 0 point in the low temperature curability of Examples.

In addition, when the blending amount of a predetermined amine compound is 0% by weight, it is understood that the evaluation point of the low temperature curability is 0, for example, no curing is yielded at 200°C or so.

Similar to the above, when the blending amount of a predetermined amine compound is increased to 6 to 7% by weight or so, the evaluation point of the low temperature curability is significantly increased, for example, 3 or so.

Furthermore, when the blending amount of a predetermined amine compound is additionally increased to 8% by weight, 10% by weight, and 14% by weight in order, the evaluation point of the low temperature curability is significantly increased, already showing the saturation, for example, indicating 5 or so.

Accordingly, from the characteristic curve shown in Fig. 2, it is understood that an aqueous solvent solution of an imide group-containing compound with desired low temperature curability can be obtained by suitably modifying the blending amount of a predetermined amine compound.

### (3) Blending amount 2

Furthermore, in the case of blending the amine compound having a predetermined boiling point, it is also preferable to have a determination by considering the blending amount of the imide group-containing compound.

More specifically, the blending amount of the amine compound is preferably set at a value within the range of from 0.1 to 20 parts by weight relative to 100 parts by weight of the imide group-containing compound.

That is because, by having a constitution with a limited blending amount of the amine compound, the viscosity increase of the aqueous solution of an imide group-containing compound can be certainly suppressed, and, at the same time, the possibility of inhibiting the reactivity of an imide group-containing compound to be obtained is reduced.

Namely, that is because, if the blending amount of the amine compound is a value of below 0.1 part by weight, the viscosity stabilizing effect of the amine compound may be significantly reduced depending on the type of the amine compound or the like.

On the other hand, that is because there is a case in which the heat resistance or mechanical properties of the amine compound to be obtained significantly deteriorates or the low temperature curability deteriorates when the blending amount of the amine compound is higher than 20 parts by weight.

As such, the blending amount of the amine compound is more preferably a value within the range of from 0.5 to 10 parts and even more preferably a value within the range of from 1 to 5 parts by weight relative to 100 parts by weight of the imide group-containing compound.

### 5. Viscosity stabilizer

### (1) Type

The type of the viscosity stabilizer is characterized in that it is an orthocarboxylic ester and a chelating agent, or either one of them.

In other words, by blending such a viscosity stabilizer, it is possible to reduce the increase in viscosity of the aqueous solvent solution of an imide group-containing compound during storage and thus to obtain an aqueous solvent solution of an imide group-containing compound which is excellent in storage stability.

In addition, more specifically, examples of the orthocarboxylic ester may include at least one dehydrating agent such as trimethyl orthoformate, triethyl orthoformate, tripropyl orthoformate tributyl orthoformate, trimethyl orthoacetate, triethyl orthoacetate, tripropyl orthoacetate, tributyl orthoacetate, trimethyl orthopropionate, triethyl orthopropionate, tripropyl orthopropionate, tributyl orthopropionate, trimethyl orthovalerate, triethyl orthovalerate, trimethyl orthochloroacetate, triethyl orthochloroacetate, tripropyl orthochloroacetate, tributyl orthochloroacetate, trimethyl orthodichloroacetate, and triethyl orthodichloroacetate.

Particularly, trimethyl orthoformate and triethyl orthoformate are a suitable orthocarboxylic ester since these esters absorb a trace amount of moisture which leads to an increase in viscosity and thus favorable viscosity stability is exhibited even when blended in a relatively small amount.

In addition, the chelating agent is preferably at least one of phytic acid, tannic acid, gallic acid, and the like.

The reason for this is because such a viscosity stabilizer can capture a free metal ion or the like and thus can effectively suppress an increase in viscosity even when the blending amount of the viscosity stabilizer is relatively small.

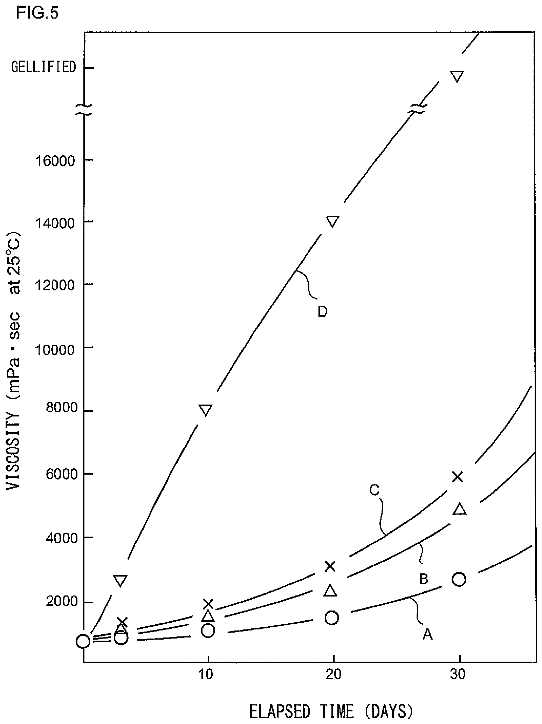

Herein, the viscosity maintaining effect (viscosity increase inhibitory effect) with regard to the aqueous solvent solution of an imide group-containing compound by various types of viscosity stabilizers will be described with reference to Fig. 5.

In Fig. 5, the horizontal axis represents number of the days (days) elapsed for the room-temperature storage of the aqueous solvent solution of an imide group-containing compound containing a viscosity stabilizer (2% by weight) based on Example 1 or the like and the vertical axis represents the viscosity (mPa·sec) of the aqueous solvent solution of an imide group-containing compound containing the viscosity stabilizer.

In addition, the characteristic curve A corresponds to the aqueous solvent solution of an imide group-containing compound in which trimethyl orthoformate is blended as a viscosity stabilizer, the characteristic curve B corresponds to the aqueous solvent solution of an imide group-containing compound in which triethyl orthoformate is blended as a viscosity stabilizer, and the characteristic curve C corresponds to the aqueous solvent solution of an imide group-containing compound in which phytic acid is blended as a viscosity stabilizer. Meanwhile, the characteristic curve D corresponds to the aqueous solvent solution of an imide group-containing compound that does not contain any viscosity stabilizer.

The viscosity of the aqueous solvent solution of an imide group-containing compounds is about 2,500 mPa·sec, 4,900 mPa·sec, and 6,100 mPa·sec, respectively, even after the elapse of 30 days although a slight increase is observed along with the elapse of days in a case in which various types of viscosity stabilizers are contained in a predetermined amount (2% by weight) as illustrated in these characteristic curves A to C in Fig 5.

On the other hand, in the case of the aqueous solvent solution of an imide group-containing compound that does not contain any viscosity stabilizer, the viscosity of the aqueous solvent solution of an imide group-containing compound increases significantly along with the elapse of days and the aqueous solvent solution of an imide group-containing compound is gelled after the elapse of 30 days, and thus a state in which the viscosity may not be measured is indicated as illustrated in the characteristic curve D.

In other words, it can be said that, compared to a case in which a viscosity stabilizer is not contained at all, an increase in viscosity during storage can be effectively suppressed by blending a predetermined viscosity stabilizer with the aqueous solvent solution of an imide group-containing compound as it can be understood from the comparison of the characteristic curves A to D in Fig.5.

### (2) Blending amount

In addition, it is preferable that the blending amount of the viscosity stabilizer is a value within the range of from 0.1 to 20 parts by weight with respect to 100 parts by weight of the imide group-containing compound.

The reason for this is because the possibility that the reactivity or the like of the imide group-containing compound to be obtained is inhibited by the viscosity stabilizer is reduced as well as an increase in viscosity can be certainly suppressed according to the constitution in which the blending amount of the viscosity stabilizer is limited within this range.

More specifically, this is because there is a case in which the viscosity stabilizing effect significantly decreases depending on the kind or the like of the viscosity stabilizer when the blending amount of the viscosity stabilizer is a value of below 0.1 part by weight.

On the other hand, this is because there is a case in which the heat resistance or mechanical properties of the polyimide film to be obtained significantly deteriorates or the low-temperature curability deteriorates when the blending amount of the viscosity stabilizer is a value of above 20 parts by weight.

Consequently, the blending amount of the viscosity stabilizer is more preferably a value within the range of from 0.2 to 10 parts and even more preferably a value within the range of from 0.5 to 5 parts by weight with respect to 100 parts by weight of the imide group-containing compound.

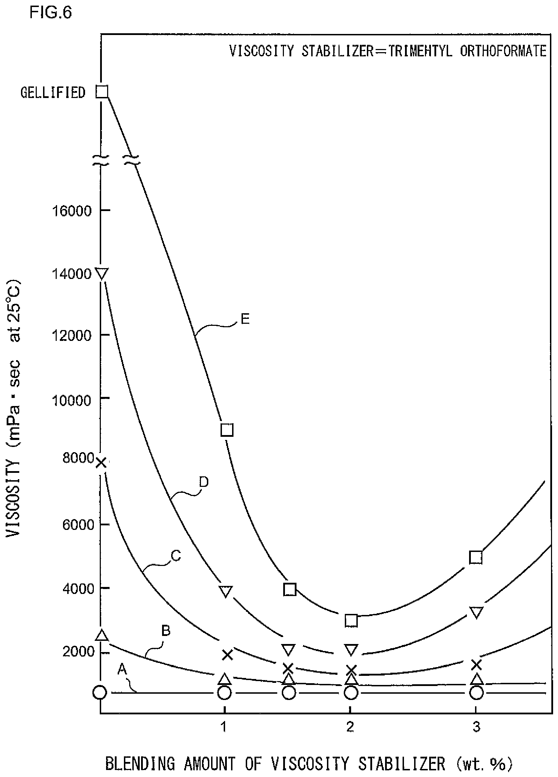

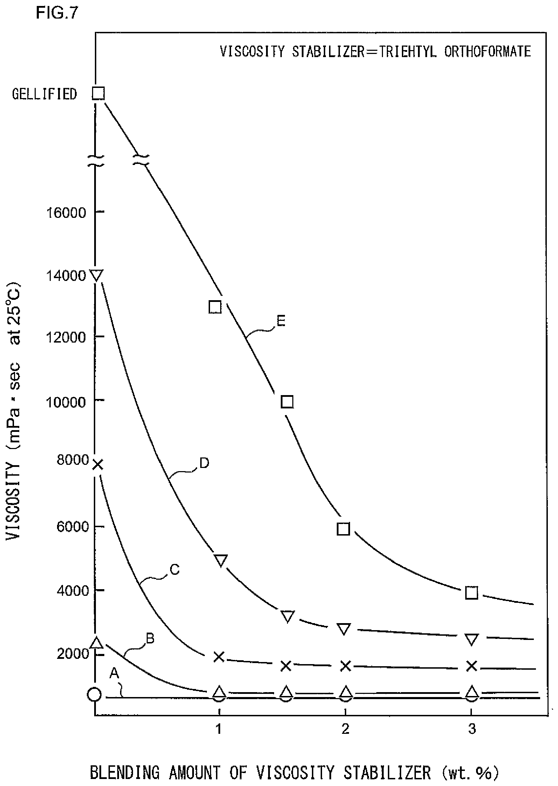

Herein, the viscosity maintaining effect with regard to the aqueous solvent solution of an imide group-containing compound by a variation in the blending amount of the viscosity stabilizer will be described with reference to Fig. 6 and Fig. 7.

In addition, in Fig. 6, the horizontal axis represents the blending amount (% by weight) of the viscosity stabilizer (trimethyl orthoformate) in the aqueous solvent solution of an imide group-containing compound based on Example 1 or the like and the vertical axis represents the viscosity (mPa·sec) of the aqueous solvent solution of an imide group-containing compound containing the viscosity stabilizer.

Moreover, the characteristic curve A corresponds to the aqueous solvent solution of an imide group-containing compound having a number of days elapsed of 0 day, that is, in the initial state. Moreover, the characteristic curve B corresponds to the aqueous solvent solution of an imide group-containing compound after the elapse of 3 days, the characteristic curve C corresponds to the aqueous solvent solution of an imide group-containing compound after the elapse of 10 days, the characteristic curve D corresponds to the aqueous solvent solution of an imide group-containing compound after the elapse of 20 days, and the characteristic curve E corresponds to the aqueous solvent solution of an imide group-containing compound after the elapse of 30 days.

The viscosity of the aqueous solvent solution of an imide group-containing compound is about 2,500 mPa·sec even after the elapse of 30 days, for example, when the blending amount of the viscosity stabilizer is 2% by weight relative to the total amount although a difference in the increase in viscosity is observed depending on the difference in the blending amount (1% by weight, 1.5% by weight, 2% by weight, and 3% by weight) of the viscosity stabilizer (trimethyl orthoformate) as illustrated in these characteristic curves A to E in Fig 6.

On the other hand, in a case in which the viscosity stabilizer is not contained at all (0% by weight), the viscosity of the aqueous solvent solution of an imide group-containing compound increases significantly along with the elapse of days, the aqueous solvent solution of an imide group-containing compound is gelled after the elapse of 30 days, and thus a state in which the viscosity may not be measured is indicated.

In other words, it can be said that, compared to a case in which the viscosity stabilizer is not contained at all, an increase in viscosity during storage can be effectively suppressed by blending a predetermined amount of viscosity stabilizer with the aqueous solvent solution of an imide group-containing compound as it can be understood from the comparison of the characteristic curves A to E in Fig. 6.

Furthermore, Fig. 7 is an example in which triethyl orthoformate is used as a kind of the viscosity stabilizer, the matters other than the type are the same as those illustrated in Fig. 6.

In other words, the viscosity of the aqueous solvent solution of an imide group-containing compound is about 6,100 mPa·sec even after the elapse of 30 days, for example, when the blending amount of the viscosity stabilizer is 2% by weight although a difference in the increase in viscosity is observed depending on the blending amount (1% by weight, 1.5% by weight, 2% by weight, and 3% by weight) of the viscosity stabilizer (triethyl orthoformate) as illustrated in these characteristic curves A to E in Fig 7, and thus it can be understood that an increase in viscosity during storage can be effectively suppressed compared with a case in which the viscosity stabilizer is not contained at all (0% by weight).

### 7. Surfactant

### (1) Type

In addition, it is preferable that a predetermined surfactant is blended in the aqueous solvent solution of an imide group-containing compound in order to improve the stability of the solution, the dispersibility of the imide group-containing compound, and the wettability of the solution to the substrate to be coated, and further, in order to adjust the surface smoothness of the coating film obtained.

Examples of such a surfactant may include an anionic surfactant, a cationic surfactant, and a nonionic surfactant, and specific examples thereof may include an ammonium salt-based surfactant, an amine salt-based surfactant, a fluorine-based surfactant, a siloxane-based surfactant, and a polymeric surfactant.

### (2) Blending amount

In addition, it is preferable that the blending amount of the surfactant is a value within the range of from 0.01 to 10% by weight relative to the total amount of the aqueous solvent solution of an imide group-containing compound in the case of blending a surfactant with the aqueous solvent solution of an imide group-containing compound.

The reason for that is because the addition effect is not exhibited in some cases when the blending amount of the surfactant is a value of below 0.01% by weight and the heat resistance or mechanical strength of the polyimide resin to be obtained decreases in some cases when the blending amount of the surfactant is a value of above 10% by weight.

As such, the blending amount of the surfactant is more preferably a value within the range of from 0.1 to 5% by weight and even more preferably a value within the range of from 0.5 to 1% by weight relative to the total amount of the aqueous solvent solution of an imide group-containing compound although it also depends on the type thereof.

In addition, it is also preferable that a colorant such as a dye or a pigment is blended according to the use or shape of the polyimide resin to be obtained.

Particularly, because the low temperature heating effect at 150°C or lower and more preferably 130°C or lower can be obtained, the thermal decomposition of the various kinds of pigments or dyes blended as an additive does not occur, and as a result, it is possible to obtain a colored polyimide film which has been impossible in a related art.

### 8. Various characteristics and uses

### (1) Low temperature curability

In addition, with regard to the low temperature curability of the imide group-containing compound to be contained in the aqueous solvent solution of an imide group-containing compound, it is preferable that a predetermined polyimide resin is obtained by thermal curing at a temperature condition of 200°C or lower, more preferably a temperature condition of 150°C or lower, and even more preferably at a temperature condition of 120°C or lower.

As such, it is preferable that a polyimide resin (imidation rate of 70% or more) which is represented by the infrared spectroscopy chart illustrated in Fig. 4 is yielded when the imide group-containing compound (compound A) represented by the infrared spectroscopy chart illustrated in Fig. 3 is treated under the normal heating conditions including a temperature of from 100°C to 200°C for 30 to 60 minutes.

More specifically, it is possible to stably form a predetermined polyimide film even for the substrate composed of an olefin resin such as polypropylene when the imide group-containing compound is thermally cured under the heating conditions of from 100°C to 150°C for 30 minutes.

In addition, if the heating conditions of from over 150°C to 200°C for 30 minutes, it is possible to form a predetermined polyimide film with respect to a metal substrate or a ceramic substrate. As a matter of course, it is also possible to stably form a predetermined polyimide film even with respect to a resin substrate composed of a polyester resin or the like.

### (2) Solubility

Furthermore, with regard to the solubility of the imide group-containing compound, it is desirable that the imide group-containing compound is dissolved sufficiently and also within a short time in an aqueous solvent such as water or alcohol.

Namely, it is preferable that, when the imide group-containing compound with an infrared spectroscopy chart shown in Fig. 3 (for example, compound A) is dissolved in water or alcohol by using a stirring device such as homomixer or planetary mixer such that the solid matter concentration is within a range of from 10 to 30% by weight, and more preferably 12 to 20% by weight, for example, a homogeneous solution is yielded within 60 minutes, and it is more preferable that a homogeneous solution is yielded within 30 minutes.

### (3) Viscosity

Furthermore, it is desirable that the viscosity of the aqueous solvent solution of an imide group-containing compound is set at a value within a range of from 10 to 500,000 mPa·sec (measurement device: Type B viscometer, measurement temperature: 25°C, ditto for the followings).

That is because, by having the viscosity of the aqueous solvent solution of an imide group-containing compound limited to a predetermined range, not only the handlability is improved but also the coating property is enhanced, and it can be uniformly and also rapidly blended with other blending components such as a thermoplastic resin component, a thermocurable resin component, a photocurable resin component, a metal material, and a ceramic material.

As such, the viscosity of the aqueous solvent solution of an imide group-containing compound more preferably is a value within the range of from 1,000 to 80,000 mPa·sec, and even more preferably is a value within the range of from 5,000 to 30,000 mPa·sec.

### (4) Use

Examples of the use of the aqueous solvent solution of an imide group-containing compound of the invention include, although not particularly limited, a heat resistant paint and an electrically insulating material.

Moreover, according to scattering of the predetermined aqueous solvent by heating at a predetermined temperature and also thermal curing at the same time, the aqueous solvent solution of an imide group-containing compound of the invention can be used as a raw material for producing a polyimide film having favorable heat resistance or the like, a casing body of a heat resistant electric part, a heat resistant material of an electronic part, a heat resistant circuit board, a heat resistant container, a heat resistance mechanical part, a heat resistant automobile part, or the like.

For example, when a separator of a lithium battery is to be fabricated, it is preferable that a paper or a non-woven fabric as a substrate is prepared and impregnated with the aqueous solvent solution of an imide group-containing compound of the invention followed by a heating treatment for 10 to 60 minutes at 150 to 200°C.

Namely, as a polyimide resin-coated separator of a lithium battery, excellent characteristics like heat resistance, lightweightness, non-flammability, durability, chemical resistance (resistance to electrolyte solution), economic value, or the like can be exhibited.

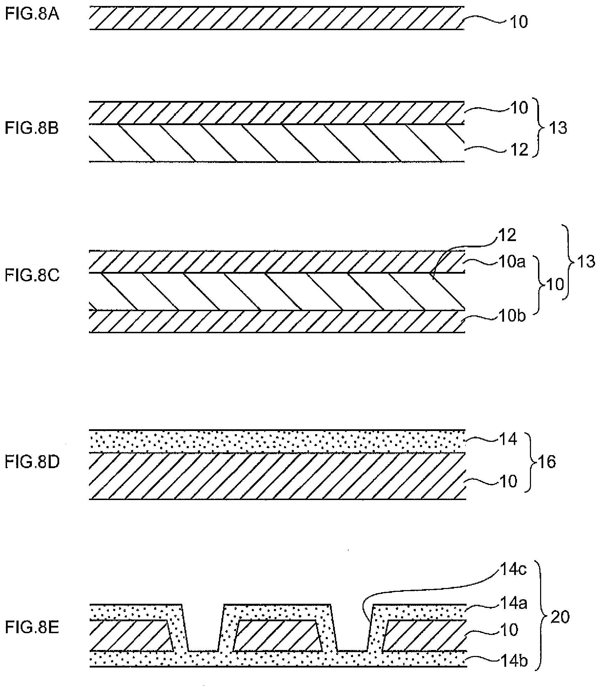

Herein, the aspects of the use of the aqueous solvent solution of an imide group-containing compound will be described more specifically with reference to Figs. 8(a) to 8(e).

First, a representative use of the aqueous solvent solution of an imide group-containing compound is a raw material of a monolayer polyimide membrane (for example, polyimide film) 10 for improving the heat resistance or electrical insulating properties as illustrated in Fig. 8(a), and it can be obtained by thermal curing at a predetermined condition while scattering at the same time the aqueous solvent from the aqueous solvent solution of an imide group-containing compound.

In addition, it is also preferably a raw material of a composite resin film 13 which is formed by layering the polyimide film 10 in order to improve the heat resistance or electrical insulating properties of another resin film 12 as illustrated in Fig. 8(b). In other words, it is also preferable to have a composite resin film 13 having heat resistance or the like that is obtained by forming the predetermined polyimide film 10 derived from the aqueous solvent solution of an imide group-containing compound of the invention on the surface of the resin film 12 such as a polyester film or a polyolefin film.

In addition, as illustrated in Fig. 8(c), it is also preferable that a composite resin film 13' is formed by layering polyimide film 10' (10a and 10b) derived from the aqueous solvent solution of an imide group-containing compound of the invention on both sides of another resin film 12, although it is a modification example of the composite resin film 13 illustrated in Fig. 8(b).

In addition, as illustrated in Fig. 8(d), it is also preferable to have an aspect that a metal composite polyimide film (it may be also referred to as a polyimide board or a TAB tape) 16 is obtained by forming a metal layer 14 on one side of the polyimide film 10 derived from the aqueous solvent solution of an imide group-containing compound of the invention.

It is possible to omit an adhesive layer between the polyimide film 10 and the metal layer 14 in the case of such a metal composite polyimide film 16 so as to contribute to the metal composite polyimide film 16 formed in a thin film.

Furthermore, as illustrated in Fig. 8(e), it is also preferable to fabricate a double-sided circuit board 20 by forming a first metal layer 14a and a second metal layer 14b on both sides of the polyimide film 10 derived from the aqueous solvent solution of an imide group-containing compound of the invention and also forming a via hole 14c which electrically connects the first metal layer 14a and the second metal layer 14b.

### [Second embodiment]

The second embodiment is a method for producing an aqueous solvent solution of an imide group-containing compound including the following step (1) to step (3):
(1) a step of preparing a polyimide molded article by cutting the polyimide molded article into a predetermined size,
(2) a step of hydrolyzing the polyimide molded article at temperature conditions of 50 to 100°C in the presence of water and a basic compound to have an imide group-containing compound in solution state, and
(3) a step of blending the imide group-containing compound with an aqueous solvent and an amine compound of which boiling point is a value within the range of from 85 to 145°C to obtain an aqueous solvent solution of an imide group-containing compound,
characterized in that the imide group-containing compound obtained from the step (2) has, in an infrared spectroscopy chart, an absorption peak at a wave number of 1,500 cm⁻¹ which is derived from a benzene ring and an absorption peak at a wave number of 1,375 cm⁻¹ which is derived from an imide group, and, at the same time, when the height of the absorption peak at a wave number of 1,500 cm⁻¹ which is derived from a benzene ring is denoted as S1 and the height of the absorption peak at a wave number of 1,375 cm⁻¹ which is derived from an imide group is denoted as S2, the ratio of S1/S2 is a value within the range of from 2 to 10.

### 1. Step (1)

Step (1) is a step of preparing a polyimide molded article by cutting the polyimide molded article into a predetermined size.

Namely, for the partial hydrolysis of the next step, a polyimide molded article which has been conventionally treated as an industrial waste is widely employed as a raw material.

Hence, examples of the suitable polyimide molded article may include a polyimide film, a polyimide paint, a polyimide-based resist, a polyimide electrical part enclosure, a polyimide electronic part material, a polyimide container, a polyimide machine part, and a polyimide automobile part.

Moreover, a composite laminate such as a circuit board having a metal circuit pattern formed on a surface of the polyimide film surface or a TAB tape can also be used as a polyimide molded article as the raw material at the time of the production of the imide group-containing compound of the invention.

Hence, it is preferable that, in the step (1), the maximum width or the average particle size of the polyimide molded article is adjusted in advance by cutting or classifying the polyimide molded article as an industrial waste by using a cutting device, a grinding device, or a classifying device.

In other words, it is preferable that the maximum width or the average particle size of the polyimide molded article is adjusted in advance by cutting or classifying the polyimide molded article as an industrial waste by using a cutter, a knife, a chopper, a shredder, a ball mill, a grinding device, a sieve, a punching metal, or a cyclone so that the partial hydrolysis thereof can be performed more uniformly and rapidly.

More specifically, the average width of the polyimide molded article is preferably 10 mm or less and more preferably a value within the range of from 1 to 5 mm in the case of adjusting to a rectangular shape.

In addition, the average width of the polyimide molded article is preferably 10 mm or less, and more preferably a value within the range of from 1 to 5 mm in the case of adjusting to a particle shape.

Furthermore, it is preferable that the polyimide molded article is introduced into a grinder for resin equipped with a perforated metal, a sieve or the like and crushed while cooling with dry ice or the like to obtain a polyimide crushed product having a small piece or granular shape in order to have a further even maximum width or average particle size.

### 2. Step (2)

Next, the step (2) is a step of partially hydrolyzing the polyimide molded article having a predetermined size in the presence of at least water and a basic compound under a temperature condition of from 40 to 100°C and more preferably from 50 to 80°C to obtain a crude imide group-containing compound.

Hence, it is preferable that the polyimide molded article is partially hydrolyzed in the presence of at least water and a basic compound at a predetermined temperature, for example, at a normal pressure, and under a condition of from 1 to 48 hours.

Herein, the basic compound means a compound which generates a hydroxide ion, and examples thereof may include a hydroxide, a carbonate, a hydrogen carbonate, and a fatty acid salt of an alkali metal or an alkaline earth metal such as sodium hydroxide, potassium hydroxide, calcium hydroxide, sodium hydrogen carbonate, potassium hydrogen carbonate, sodium acetate, potassium acetate, and sodium propionate or ammonia, ammonium carbonate, ammonium hydrogen carbonate, ammonium acetate, hydroxylamine, hydrazine, tetramethyl ammonium hydroxide, tetraethyl ammonium hydroxide, and an organic amine compound.

Particularly, sodium hydroxide or potassium hydroxide is preferably used since the hydrolysis proceeds at a relatively low temperature and in a mild manner.

Meanwhile, as described above, it is possible to confirm whether the polyimide molded article is partially hydrolyzed and thus the desired crude imide group-containing compound is obtained or not by determining the presence or absence of the absorption peak derived from an imide group, the absorption peak derived from an amide group, the absorption peak derived from a carboxyl group or the like in the infrared spectroscopy chart obtained by the infrared spectroscopic measurement, the height of each of the peaks, and further the fact that the relative height ratio thereof or the like to the absorption peak derived from a benzene ring is a numeral value within a predetermined range.

### 3. Step (3)

Next, the process (3) is a process of purifying the crude imide group-containing compound to obtain an imide group-containing compound having an absorption peak derived from an imide group at a wavenumber of 1,375 cm⁻¹, an absorption peak derived from an amide group at a wavenumber of 1,600 cm⁻¹, and an absorption peak derived from a carboxyl group at a wavenumber of 1,413 cm⁻¹ in the infrared spectroscopy chart obtained by the infrared spectroscopic measurement.

Hence, it is preferable to prepare an imide group-containing compound from which impurities are removed, for example, by repeatedly performing a series of processes of an acid treatment (for example, treatment with hydrochloric acid, nitric acid, or sulfuric acid, phosphoric acid, organic acid, or the like), washing with water, an alkali treatment (sodium hydroxide, potassium hydroxide, sodium carbonate, or potassium carbonate treatment, or the like), washing with water, and drying from 1 to 10 times to purify the crude imide group-containing compound.

In addition, in order to avoid effectively the influence of an acid component or an alkali component remaining in the crude imide group-containing compound, for example, the influence on heat resistance or metal corrosion or the like, it is more preferable to use phosphoric acid, which is a weak acid, for the acid treatment, and it is more preferable to use potassium hydroxide for the alkali treatment.

Moreover, to have the same particle size after purification and drying of the crude imide group-containing compound, it is preferable to perform, by using a sieve, size classification of the imide group-containing compound for each predetermined particle size.

Meanwhile, it is possible to confirm whether the crude imide group-containing compound is sufficiently purified or the like by the fact that the content of the element (or an ion of element) such as chlorine, sulfur, phosphorus, aluminum, or magnesium in the purified crude imide group-containing compound is equal to or less than a predetermined amount confirmed by the quantification of the element using the ion chromatography or the X-ray photoelectron spectroscopy (XPS).

More specifically, the purification degree of the crude imide group-containing compound can be determined, for example, based on the fact that the quantity of the chloride ion is 100 ppm or less, more preferably 10 ppm or less, and even more preferably 1 ppm or less as confirmed by the quantification of the chloride ion using the ion chromatography for elemental analysis.

Furthermore, the purified product of a crude imide group-containing compound may be also referred to as a partial hydrolyzate.

In addition, the purified product of the crude imide group-containing compound is a representative example of a partial hydrolyzate of the polyimide molded article having a predetermined structure, as the infrared spectroscopy chart is illustrated in Fig. 3.

Namely, it is an imide group-containing compound obtained by partially hydrolyzing a polyimide molded article having a predetermined size in the presence of water and a basic compound under a temperature condition of from 50 to 100°C even though it is an example, and the imide group-containing compound which has a predetermined structure and is represented by the aforementioned formula (1) is the subject.

Furthermore, production of the imide group-containing compound which has a predetermined structure and is represented by the formula (1) can be determined by carrying out an infrared spectroscopy analysis.

As such, by having at least an imide group, an amide group, and a carboxyl group, and further a carbonyl group or the like in the molecule composed of a carbon atom, it is possible to obtain an imide group-containing compound which is curable at low temperature and exhibits excellent solubility in various kinds of aqueous solvents or adhesiveness to various adherends.

### 4. Step (4)

Finally, the step (4) is a step for obtaining an aqueous solvent solution of an imide group-containing compound with predetermined concentration by dissolving the imide group-containing compound obtained from the step (3) in an aqueous solvent.

More specifically, it is preferable to obtain an aqueous solvent solution of an imide group-containing compound by using at least one of known stirring devices like a propeller mixer, a homomixer, a planetary mixer, a ball mill, a jet mill, a vibration mill, and a three-rolls and dissolving basically the imide group-containing compound obtained from the step (3) in a predetermined amount of an aqueous solvent.

Furthermore, although it is desirable to use a particle-shape product having a predetermined average particle diameter (for example, 0.1 to 100 µm) as the imide group-containing compound obtained in the step (3) and dissolve it in an aqueous solvent, it is also desirable to use an imide group-containing compound in paste state, semi-solid state, or solid state in which the crude imide group-containing compound remains in a purified state.

In the step (3), processing the imide group-containing compound into a particle-shape product having a predetermined average particle diameter (for example, 0.1 to 100 µm) requires, other than the purification step, several steps like dehydration treatment or low temperature drying treatment.

On the contrary, the imide group-containing compound in paste state, semi-solid state, or solid state in which the crude imide group-containing compound remains in a purified state can be dissolved uniformly within a short time in an aqueous solvent even though the water content is 20 to 60% by weight in the total amount.

As such, the production cost and production time can be dramatically saved (for example, 1/3 to 1/5), and, at the same time, incorporation of contaminating materials can be effectively prevented during the production.

Meanwhile, aside from the discussion regarding the use of the imide group-containing compound as a particle-shaped product or the imide group-containing compound in paste state, semi-solid state, or solid state, the blending amount (content) of the aqueous solvent is preferably set at a value that is within a range of from 20 to 99% by weight relative to the whole amount of the aqueous solvent solution of an imide group-containing compound.

That is because, by adjusting the blending amount of the aqueous solvent to a predetermined range, not only the handlability is improved but also the coating and drying becomes easier, and it can be uniformly and also rapidly blended with other blending components such as a thermoplastic resin component, a thermocurable resin component, a photocurable resin component, a metal material, and a ceramic material.

More specifically, that is because, when the blending amount of the aqueous solvent is a value of below 20% by weight, there is a case in which the handlability is significantly impaired due to insufficient solubility of the imide group-containing compound.

On the other hand, that is because, when the blending amount of the aqueous solvent is a value of above 99% by weight, there is a case in which the viscosity of the aqueous solvent solution of an imide group-containing compound to be obtained is excessively reduced so that it becomes difficult to form an even polyimide film having predetermined film thickness or precipitates may easily occur.

As such, relative to the whole amount of the aqueous solvent solution of an imide group-containing compound, the blending amount of the aqueous solvent more preferably is a value within the range of from 50 to 90% by weight, and even more preferably a value within the range of from 60 to 80% by weight.

Other than the above, it is desirable to blend a pseudo cross-linking inhibitor in the step (4).

Namely, while the imide group-containing compound contains a predetermined carboxyl group or hydroxyl group, the aqueous solvent solution of the imide group-containing compound contains inevitable metal ions at the same time.

As such, there may be a case in which it is present in a pseudo cross-linking state which is considered to be caused by a predetermined carboxyl group or a hydroxyl group.

In such case, a phytic acid compound, a dacuro compound, or EDTA is preferably added, either after having a pseudo cross-linking state or before having a pseudo cross-linking state to prevent a pseudo cross-linking state.

Namely, it is known that each of the phytic acid compound, which is myo-inositol hexa phophoric acid ester represented by the molecular formula of C₆H₁₈O₂₄P₆, the pholyphenol compound represented by the molecular formula of C₇₆H₅₂O₄₆, or EDTA (ethylene diamine tetraacetic acid) can capture various metal ions (for example, iron, zinc, or the like) and chelate them.

Thus, by blending the pseudo cross-linking inhibitor, metal ions that are considered to be cause of having pseudo cross-linking derived from a carboxyl group or a hydroxyl group are captured and chelated and the aqueous solvent solution of an imide group-containing compound of which viscosity may not be measured due to the pseudo cross-linking state can be modified to a coatable state with low viscosity (100 to 100,000 mPa • sec, measurement temperature: 20°C).

As such, relative to 100 parts by weight of the imide group-containing compound (before pseudo cross-linking state), the blending amount of the pseudo cross-linking inhibitor preferably is a value within the range of from 0.01 to 10 parts by weight, more preferably a value within the range of from 0.05 to 5 parts by weight, and even more preferably a value within the range of from 0.1 to 1 part by weight.

Furthermore, it is possible to blend various kinds of additives within a range in which the properties of the aqueous solvent solution of an imide group-containing compound are not impaired.

In other words, it is preferable to blend at least one kind of a polymer resin or an oligomer resin such as a fluoro resin, an epoxy resin, a phenol resin, a silicone resin, a urethane resin, an olefin resin (including an acrylic resin), a polyester resin, a polyamide resin, or a carbon resin.

In particular, by blending a fluoro resin, the gliding property or water repellency of a polyimide resin to be obtained can be enhanced.

In addition, it is also preferable to blend at least one kind of a colorant like a dye and a pigment, an electrically conductive material, an electrically insulating material, an ultraviolet absorber, a radiation-absorbing agent, a cross-linking agent, a viscosity modifier, a matting agent, a weight saving material, a fiber and the like according to the use or shape of the polyimide resin to be obtained.

### Examples

Hereinbelow, the invention will be described in greater detail based on Examples.

### [Example 1]

### 1. Production of aqueous solvent solution of an imide group-containing compound

### (1) Step 1

The KAPTON film (for example, mixed product with other KAPTON films although KAPTON-100H is the main constituent, manufactured by DU PONT-TORAY CO., LTD.) as the polyimide molded article was cut into a rectangular shape having a width of 10 mm or less using a chopper.

Next, the cut polyimide molded article was introduced into a grinder for resin (Model No. P-1314 manufactured by Horai Co., Ltd.) equipped with a punching metal having a diameter of 3 mm and the cut polyimide molded article (average particle size: about 3 mm) passing through the punching metal was crushed while cooling with dry ice added so as to obtain a crushed polyimide product as the subject of the partial hydrolysis.

### (2) Step 2

Next, into a 1,000 ml vessel equipped with a stirrer, 5 g of the crushed polyimide product thus obtained, 400 g of ion exchanged water, and 2 g of potassium hydroxide as a basic substance were introduced.

Subsequently, the inner temperature of the vessel was raised to 50°C and then the hydrolysis treatment was carried out under a condition of 24 hours while stirring the introduced materials, thereby obtaining a hydrolysis treatment solution containing a crude imide group-containing compound.

### (3) Step 3

Subsequently, an acid treatment (phosphoric acid), washing with water, an alkali treatment, and washing with water of the hydrolysis treatment solution containing the crude imide group-containing compound was repeatedly carried out five times in order to purify the crude imide group-containing compound.

Incidentally, only a dehydration press step was carried out for the purified imide group-containing compound without performing a drying step at low temperature to yield an imide group-containing compound in semi-solid state of which water content is 50% by weight in the whole amount.

Furthermore, it was confirmed by the quantitative analysis that, in the imide group-containing compound, about 0.2% by weight of potassium, about 0.02% by weight of Si, about 0.02% by weight of Ca, and 0.005% by weight of Fe were contained, respectively.

### (4) Step 4

Subsequently, into a vessel equipped with a stirrer, 493 g of ion exchange water and 90 g of N,N-dimethylaminoethanol (referred to as TYP1 in Table 2, and it is 9% by weight relative to the whole amount) were introduced and heated to 60°C under stirring.

Subsequently, 400 g of an imide group-containing compound in amorphous or semi-solid state and 10 g of trimethyl orthoformate were added to a vessel provided with a stirrer, which is maintained at 60°C, and they were heated till to have homogeneity.

Finally, 7 g of a surfactant were added to the vessel provided with a stirrer to obtain an aqueous solvent solution of an imide group-containing compound containing an imide group-containing compound (solid matter concentration: 20% by weight).

### 2. Evaluation of an imide group-containing compound and aqueous solvent solution of an imide group-containing compound

### (1) FT-IR Analysis (Evaluation 1)

By using the obtained aqueous solvent solution of an imide group-containing compound, an imide group-containing compound film with thickness of 10 µm was prepared according to a coating method.

Subsequently, by using an infrared spectrophotometer (FT-IR), presence of various functional groups (for example, imide group, amide group, carboxyl group, carbonyl group, benzene ring, and the like) was determined according to an ATR method.

Furthermore, in Fig. 3, the infrared spectroscopy chart of the obtained imide group-containing compound is shown, and in Fig. 4, the infrared spectroscopy chart of the cured product of the imide group-containing compound (conditions for thermal curing; 150°C, 30 minutes), for example, infrared spectroscopy chart of polyimide, is shown.

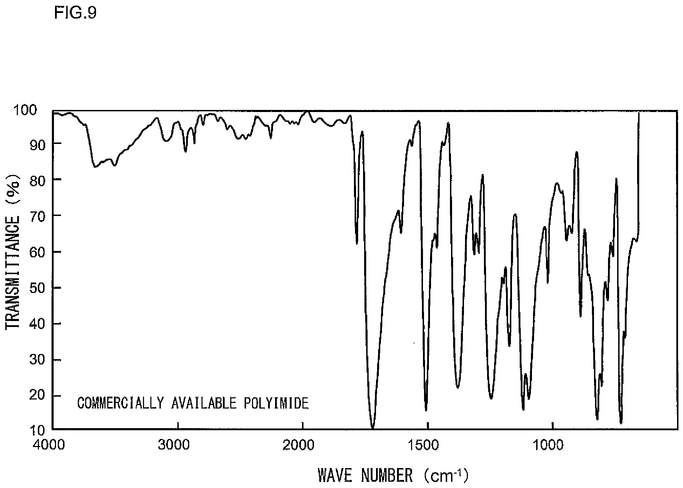

Furthermore, in Fig. 9, the infrared spectroscopy chart of a commercially available polyimide (KAPTON H) is shown for reference.

### (2) Solubility (Evaluation 2)

By using a stirring device (for example, homomixer), the obtained imide group-containing compound solution in amorphous or semi-solid state was dissolved in a mixture solution of water/N,N-dimethylaminoethanol (weight ratio = 90/10) heated to 60°C such that the solid matter concentration is 15% by weight, and then the solubility of the imide group-containing compound was evaluated in accordance with the following criteria.
⊙ (Very Good): It is dissolvable within 30 minutes.
O(Good): It is dissolvable within 60 minutes.
Δ (Fair): It is dissolvable within 120 minutes.
× (Bad): It is not dissolvable even after 120 minutes.

### (3) Low temperature curability (Evaluation 3)

The obtained aqueous solvent solution of an imide group-containing compound was coated on a soft steel iron plate (length of 80 mm, width of 30 mm, and thickness of 1 mm), and cured under the heating condition at 120°C for 30 minutes and the heating condition at 150°C for 30 minutes, respectively, to form a polyimide film having a thickness of 20 µm followed by acceleration. The low temperature curability thereof was evaluated in accordance with the following criteria.
⊙ (Very Good) A firm polyimide film is formable at 120°C and 150°C for 30 minutes, respectively.
O(Good): The polyimide film formed at 120°C for 30 minutes is slightly soft but a firm polyimide film is formable at 150°C for 30 minutes.
Δ (Fair): The polyimide film formed at 120°C for 30 minutes is slightly soft but an almost firm polyimide film is formable at 150°C for 30 minutes.
× (Bad): Due to insufficient curing, a firm polyimide film is not formed at both 120°C and 150°C for 30 minutes.

### (4) Adhesiveness (Evaluation 4)

The crosscut adhesion test of the polyimide film (a product cured at 150°C for 30 minutes) obtained in the evaluation on the low temperature curability was carried out in accordance with JIS K-5400, and the adhesiveness was evaluated in accordance with the following criteria. ⊙ (Very Good): It is a crosscut having a number of peeling of 0/100.
○ (Good): It is a crosscut having a number of peeling of from 1 to 5/100.
Δ (Fair): It is a crosscut having a number of peeling of from 6 to 10/100.
× (Bad): It is a crosscut having a number of peeling of 11/100 or more.

### (5) Heat resistance (Evaluation 5)

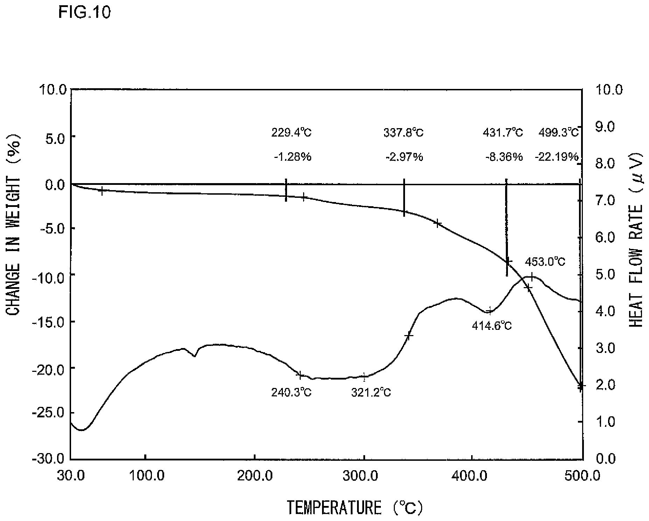

The differential scanning thermogravimetry chart (TG-DTA curve) of the polyimide film (a product cured at 150°C for 30 minutes) obtained in the evaluation on the low temperature curability was obtained as the heat resistance by heating (temperature rising rate of 10°C/min) from 30 to 500°C in nitrogen using a differential scanning thermogravimetry (TG-DTA) as illustrated in Fig. 10. Thereafter, the evaluation on the heat resistance of the polyimide film was carried out according to the following criteria based on the TG curve of the TG-DTA curve.
⊙ (Very Good): 10% weight loss temperature is 450°C or higher.
O(Good): 10% weight loss temperature is 400°C or higher.
Δ (Fair): 10% weight loss temperature is 350°C or higher.
× (Bad): 10% weight loss temperature is below 350°C.

### [Example 2]

In Example 2, the solubility and the like of the imide group-containing compound were evaluated in the same manner as in Example 1 except that the aqueous solvent solution of an imide group-containing compound containing imide group-containing compound (compound B) was obtained by changing the hydrolysis time to 36 hours and the degree of hydrolysis of the polyimide molded article as well as the kind of the KAPTON film to KAPTON H (manufactured by DU PONT-TORAY CO., LTD.) as the polyimide molded article.

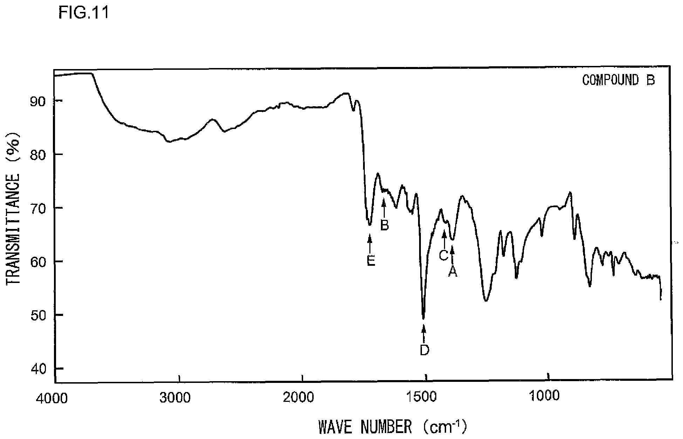

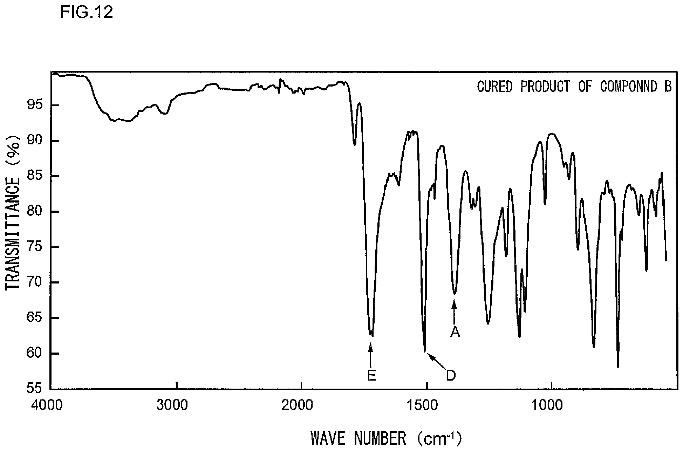

Furthermore, in Fig. 11, the infrared spectroscopy chart of the obtained imide group-containing compound is shown, and in Fig. 12, the infrared spectroscopy chart of the cured product of the imide group-containing compound (conditions for thermal curing; 150°C, 30 minutes), for example, infrared spectroscopy chart of polyimide, is shown.

### [Example 3]

In Example 3, the solubility and the like of the imide group-containing compound were evaluated in the same manner as in Example 1 except that the aqueous solvent solution of an imide group-containing compound containing imide group-containing compound (compound C) was obtained by shortening the hydrolysis time to 12 hours and changing the degree of hydrolysis of the polyimide molded article as well as changing the kind of the KAPTON film to KAPTON EN (manufactured by DU PONT-TORAY CO., LTD.) as the polyimide molded article.

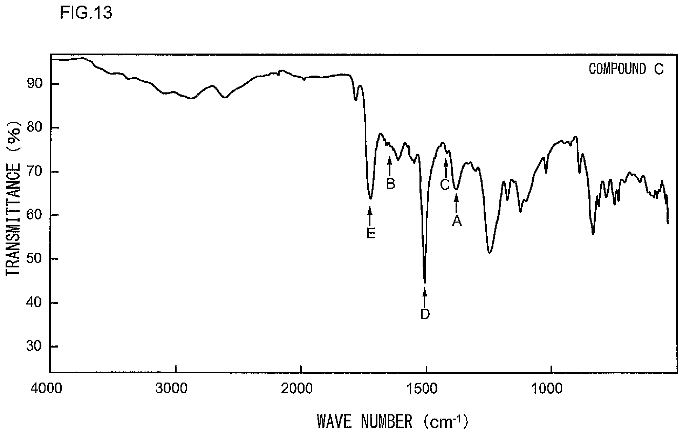

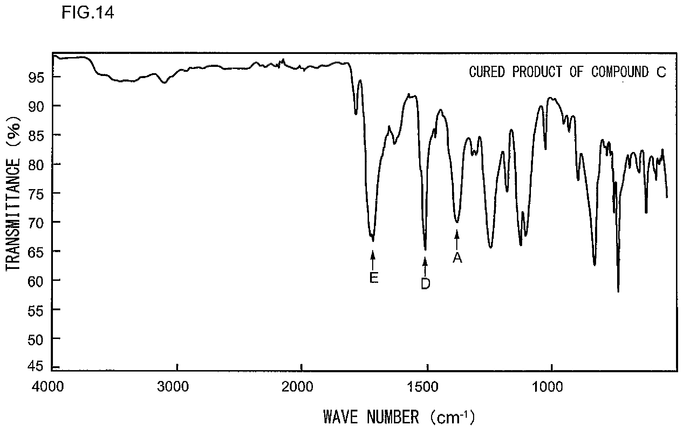

Furthermore, in Fig. 13, the infrared spectroscopy chart of the obtained imide group-containing compound is shown, and in Fig. 14, the infrared spectroscopy chart of the cured product of the imide group-containing compound (conditions for thermal curing; 150°C, 30 minutes), for example, infrared spectroscopy chart of polyimide, is shown.

### [Examples 4 to 6]

In Examples 4 to 6, evaluation of the aqueous solvent solution of an imide group-containing compound was carried out in the same manner as in Example 1 except that the blending amount of N,N-dimethylaminoethanol (% by weight) was changed to 8% by weight, 7% by weight, and 6% by weight, respectively, relative to the whole amount.

### [Examples 7 to 10]

In Examples 7 to 10, the aqueous solvent solution of an imide group-containing compound was prepared in the same manner as in Example 1 except that, instead of N,N-dimethylaminoethanol, triethylamine (boiling point: 89.5°C, Example 7, referred to as TYP2 in Table 2), N-methylmospholine (115°C, Example 8, referred to as TYP3 in Table 2), N,N,N',N'-tetramethylethylenediamine (121°C, Example 9, referred to as TYP4 in Table 2), and N,N-diethylethanolamine (boiling point: 134°C, Example 10, referred to as TYP5 in Table 2) are used, respectively, followed by evaluation.

### [Comparative Example 1]

In Comparative Example 1, each of the solubility, low temperature curability, and adhesiveness was evaluated in the same manner as in Example 1 except that sodium hydroxide was added to the polyimide molded article in a molar amount of about 80 times the theoretical decomposition amount (about 66 g) and then the hydrolysis was carried out under the condition of 80°C, a normal pressure, and 7 days, and further, the resultant was neutralized with an acidic substance so as to completely decompose to pyromellitic acid and an aromatic amine.

However, because a film may not be formed by curing, actually, the test relating to low temperature curability, adhesiveness or the like were incomplete.

### [Comparative Example 2]

In Comparative Example 2, each of the solubility, low temperature curability, and adhesiveness was evaluated in the same manner as in Example 1 except that potassium hydroxide was added to the polyimide molded article in a molar amount of 1 time the theoretical decomposition amount (about 0.1 g) and then the hydrolysis was carried out under the condition of 30°C, a normal pressure, and 8 hours, and further, the resultant was neutralized with an acidic substance and also purified so as to obtain a compound (compound D) that is almost not partially hydrolyzed.

However, because it was not actually possible to form a uniform film by dissolution, the test relating to low temperature curability, adhesiveness or the like was incomplete.

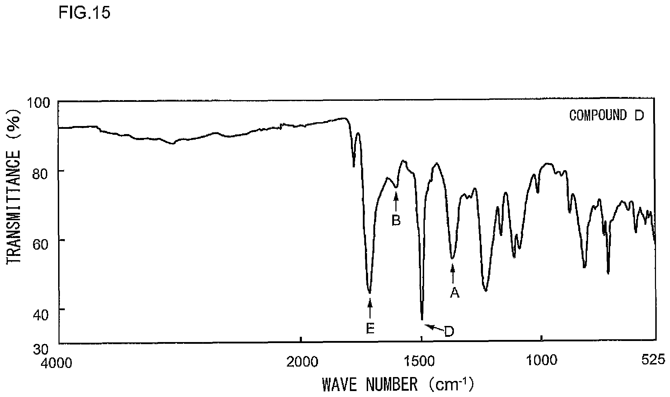

Furthermore, the infrared spectroscopy chart of the obtained imide group-containing compound (compound D) is shown in Fig. 15.

### [Comparative Examples 3 and 4]

In Comparative Examples 3 and 4, the aqueous solvent solutions of an imide group-containing compound were prepared and evaluated in the same manner as in Examples 1 and 2 except that N,N-dimethylaminoethanol as a predetermined amine compound was not blended at all.

However, because it was not actually possible to form a uniform film by dissolution, the test relating to low temperature curability, adhesiveness or the like was incomplete.

**Table 1**

| | Polyimide | Water (g) | Basic compound (g) | Hydrolysis temperature (°C) | Hydrolysis time (Hrs) | Shape |
|---|---|---|---|---|---|---|
| Example 1 | 100H | 400 | 2 | 50 | 24 | Amorphous/semi-solid state |
| | | | KOH | | | |
| Example 2 | H | 400 | 2 | 50 | 36 | Amorphous/semi-solid state |
| | | | KOH | | | |
| Example 3 | EN | 400 | 2 | 50 | 12 | Amorphous/semi-solid state |
| | | | KOH | | | |
| Example 4 to 6 | 100H | 400 | 2 | 50 | 24 | Amorphous/semi-solid state |
| | | | KOH | | | |
| Example 7 to 10 | 100H | 400 | 2 | 50 | 24 | Amorphous/semi-solid state |
| | | | KOH | | | |
| Comparative Example 1 | 100H | 400 | 66 | 80 | 168 | Amorphous/semi-solid state |
| | | | NaOH | | | |
| Comparative Example 2 | 100H | 400 | 0.1 | 30 | 8 | Amorphous/semi-solid state |
| | | | KOH | | | |
| Comparative Example 3 | 100H | 400 | 2 | 30 | 8 | Amorphous/semi-solid state |
| | | | KOH | | | |
| Comparative Example 4 | H | 400 | 2 | 30 | 8 | Amorphous/semi-solid state |
| | | | KOH | | | |
| Comparative Example 5 | EN | 400 | 2 | 30 | 8 | Amorphous/semi-solid state |
| | | | KOH | | | |

**Table 2**

| | Basic compound | FT-IR evaluation | | | | Evaluation 2 | Evaluatio n 3 | Evaluation 4 | Evaluation 5 |
|---|---|---|---|---|---|---|---|---|---|
| | | Imide group | Amide group | Carboxyl group | Carbonyl group | | | | |
| Example 1 | TYP1 9wt.% | Present | Present | Present | Present | ⊙ | ⊙ | ⊙ | ⊙ |
| Example 2 | TYP1 9wt.% | Present | Present | Present | Present | ⊙ | ⊙ | ⊙ | ○ |
| Example 3 | TYP1 9wt.% | Present | Present | Present | Present | ⊙ | ⊙ | ○ | ○ |
| Example 4 | TYP1 8wt.% | Present | Present | Present | Present | ⊙ | ⊙ | ⊙ | ⊙ |
| Example 5 | TYP1 7wt.% | Present | Present | Present | Present | ⊙ | ○ | ⊙ | ⊙ |
| Example 6 | TYP1 6wt.% | Present | Present | Present | Present | ⊙ | ○ | ⊙ | ⊙ |
| Example 7 | TYP2 9wt.% | Present | Present | Present | Present | ○ | ⊙ | ⊙ | ⊙ |
| Example 8 | TYP3 9wt.% | Present | Present | Present | Present | ⊙ | ⊙ | ⊙ | ⊙ |
| Example 9 | TYP4 9wt.% | Present | Present | Present | Present | ⊙ | ⊙ | ⊙ | ⊙ |
| Example 10 | TYP5 9wt.% | Present | Present | Present | Present | ○ | ⊙ | ⊙ | ⊙ |
| Comparative Example 1 | TYP1 9wt.% | Absent | Absent | Present | Absent | ⊙ | × | ⊙ | × |
| Comparative Example 2 | TYP1 9wt.% | Present | Present | Absent | Present | × | × | × | × |
| Comparative Example 3 | 0wt.% | Present | Present | Present | Present | × | × | × | × |
| | | | | | | ∼Δ | ∼Δ | ∼Δ | ∼Δ |
| Comparative Example 4 | 0wt. % | Present | Present | Present | Present | × | × | × | × |
| | | | | | | ∼Δ | ∼Δ | ∼Δ | ∼Δ |

| | | | | | | | | | |
|---|---|---|---|---|---|---|---|---|---|
| Evaluation 2: solubility Evaluation 3: low temperature curability Evaluation 4: adhesiveness Evaluation 5: heat resistance | | | | | | | | | |

### [Example 11]

In Example 11, the aqueous solvent solution of an imide group-containing compound, which contains the imide group-containing compound (compound A) after purification as obtained in Example 1, was coated on an aluminum foil using a bar coater, and then thermally cured under the condition of 150°C and 30 minutes so as to form a composite film of a polyimide having a thickness of 10 µm and a metal foil having a thickness of 20 µm, and the evaluation on the appearance thereof was carried out.

As a result, it was confirmed that a polyimide excellent in transparency was firmly formed on an aluminum foil although it was colored in light yellow.

### [Example 12]

In Example 12, the aqueous solvent solution of an imide group-containing compound, which contains the imide group-containing compound (compound A) after purification as obtained in Example 1, was coated on a polyester film using a bar coater, and then thermally cured under the condition of 150°C and 30 minutes so as to form a composite film of a polyimide having a thickness of 10 µm and a polyester film having a thickness of 50 µm, and the evaluation on the appearance thereof was carried out.

As a result, it was confirmed that a polyimide excellent in transparency was firmly formed on a polyester film although it was colored in light yellow.

Furthermore, as a result of evaluating the flame retardancy based on the method of JIS L 1091 A-1, it was confirmed that the requirement thereof is satisfied.

### [Example 13]

In Example 13, 100% by weight of the aqueous solvent solution of an imide group-containing compound, which contains the imide group-containing compound (compound A) after purification as obtained in Example 1, were blended with a fluoro resin (PTFE) such that the fluoro resin is 5% by weight. After that, the resultant was coated on an iron plate using a bar coater, and then thermally cured under the heating condition at 150°C for 30 minutes so as to form a film of a polyimide having a thickness of 10 µm, and then a touch feel evaluation or the like was carried out.

As a result, it was confirmed that a uniform polyimide film having favorable surface gliding property and excellent water repellency is formed.

### [Example 14]

In Example 14, 100% by weight of the aqueous solvent solution of an imide group-containing compound, which contains the imide group-containing compound (compound A) after purification as obtained in Example 1, were blended with a fluoro resin (PTFE) such that the fluoro resin is 20% by weight, and also blended with 1% by weight of a fluorine-based surfactant at the same time. After that, the resultant was coated on an iron plate using a bar coater, and then thermally cured under the heating condition at 150°C for 30 minutes so as to form a film of a polyimide having a thickness of 10 µm, and then a touch feel evaluation or the like was carried out.

As a result, it was confirmed that a uniform polyimide film having more favorable surface gliding property and excellent water repellency is formed.

### INDUSTRIAL APPLICABILITY

As it has been explained in the above, according to partial hydrolysis of a polyimide molded article as an industrial waste or the like and, at the same time, dissolving of an imide group-containing compound having a specific structure, which has a predetermined infrared absorption peak in an infrared spectroscopy chart obtained from an infrared spectroscopy measurement, in an aqueous solvent and an amine compound having a predetermined boiling point, favorable low temperature curability, favorable heat resistance, favorable solubility in an aqueous solvent, and also a favorable film forming property or adhesiveness or the like can be obtained.

Furthermore, with this imide group-containing compound derived from a predetermined aqueous solvent solution, a predetermined aqueous solvent solution can be prepared in an amorphous state (bulk state) and a semi-solid state within a very short time by a simplified process without preparing the predetermined aqueous solvent solution in a particle shape.

Furthermore, from the viewpoint that an aqueous solvent like water can be used, excellent safety or waste treatment property is obtained, and there is also an advantage that, compared to NMP or the like, it hardly remains as residuals when a polyimide resin is prepared and heated.

As such, it was found that, a polyimide resin having heat resistance or the like equivalent to those of a related art can be obtained at a cost which is 1/10 to 1/30 or less of the cost for producing the polyimide resin of a related art.

As such, it is expected that the obtained aqueous solvent solution of an imide group-containing compound is combined with a polyimide film, a polyimide paint, or the like which have excellent heat resistance or adhesiveness and also various substrates (paper, non-woven fabric, nanofiber fibers, felt, wood, and ceramic material), and desirably used for each of various uses of a polyimide resin including an electric part enclosure, an electronic part material, a separator for Li battery, a heat resistant container, a mechanical part, and an automobile part.

In particular, because thermal curing at low temperature like 200°C or lower and more preferably at 150°C or lower is possible, thermal decomposition is not yielded even after compositing with a polyester resin, a polyolefin resin, a polyurethane resin or the like or blending with various pigments or dyes, and thus it also becomes possible to prepare a color polyimide resin which remained impossible before.

### EXPLANATIONS OF LETTERS OR NUMERALS

- 10:: polyimide (polyimide film, polyimide film)
- 12:: another resin film
- 13:: composite resin film
- 14:: metal layer
- 14a:: first metal layer
- 14b:: second metal layer
- 14c:: via hole
- 16:: metal composite polyimide film and
- 20:: double-sided circuit board

## Claims

1. An aqueous solvent solution of a compound containing an imide group obtained by partial hydrolysis of a polyimide molded article, an aqueous solvent, and an amine compound of which boiling point is 85 to 145°C, in which the imide group-containing compound has, in an infrared spectroscopy chart, an absorption peak at a wave number of 1,500 cm⁻¹ which is derived from a benzene ring and an absorption peak at a wave number of 1,375 cm⁻¹ which is derived from an imide group, and, at the same time, when the height of the absorption peak at a wave number of 1,500 cm⁻¹ which is derived from a benzene ring is denoted as S1 and the height of the absorption peak at a wave number of 1,375 cm⁻¹ which is derived from an imide group is denoted as S2, the ratio of S1/S2 is a value within the range of from 2 to 10.

2. The aqueous solvent solution of the compound containing an imide group according to claim 1, wherein the aqueous solvent is water and an alcohol compound, or either one of them.

3. The aqueous solvent solution of the compound containing an imide group according to claim 1 or 2, wherein, in the infrared spectroscopy chart of the imide group-containing compound, the aqueous solvent solution of an imide group-containing compound has an absorption peak at a wave number of 1,600 cm⁻¹ which is derived from an amide group and the ratio of S1/S3 is a value within the range of from 2 to 20 when the height of the absorption peak derived from a benzene ring at a wave number of 1,500 cm⁻¹ is denoted as S1 and the height of the absorption peak derived from an amide group at a wave number of 1,600 cm⁻¹ is denoted as S3.

4. The aqueous solvent solution of the compound containing an imide group according to any one of claims 1 to 3, wherein, in the infrared spectroscopy chart of the imide group-containing compound, the aqueous solvent solution of an imide group-containing compound has an absorption peak at a wave number of 1,413 cm⁻¹ which is derived from a carboxyl group and the ratio of S1/S4 is a value within the range of from 8 to 30 when the height of the absorption peak derived from a benzene ring at a wave number of 1,500 cm⁻¹ is denoted as S1 and the height of the absorption peak derived from a carboxyl group at a wave number of 1,413 cm⁻¹ is denoted as S4.

5. The aqueous solvent solution of the compound containing an imide group according to any one of claims 1 to 4, wherein the blending amount of the water is a value within the range of from 20 to 99% by weight relative to the whole amount.

6. The aqueous solvent solution of the compound containing an imide group according to any one of claims 1 to 5, wherein the blending amount of the amine compound is a value within the range of from 0.1 to 25% by weight relative to the whole amount.

7. The aqueous solvent solution of the compound containing an imide group according to any one of claims 1 to 6, wherein a viscosity stabilizer is blended and, at the same time, the blending amount of the viscosity stabilizer is a value within the range of from 0.1 to 10% by weight relative to the whole amount of the compound containing an imide group.

8. A method for producing an aqueous solvent solution of an imide group-containing compound including following step (1) to step (3) :
(1) a step of preparing a polyimide molded article by cutting the polyimide molded article into a predetermined size,
(2) a step of hydrolyzing the polyimide molded article at temperature conditions of 50 to 100°C in the presence of water and a basic compound to have an imide group-containing compound in solution state, and
(3) a step of blending the imide group-containing compound with an aqueous solvent and an amine compound of which boiling point is 85 to 145°C to obtain an aqueous solvent solution of an imide group-containing compound,
in which the imide group-containing compound obtained from the step (2) has, in an infrared spectroscopy chart, an absorption peak at a wave number of 1,500 cm⁻¹ which is derived from a benzene ring and an absorption peak at a wave number of 1,375 cm⁻¹ which is derived from an imide group, and, at the same time, when the height of the absorption peak at a wave number of 1,500 cm⁻¹ which is derived from a benzene ring is denoted as S1 and the height of the absorption peak at a wave number of 1,375 cm⁻¹ which is derived from an imide group is denoted as S2, the ratio of S1/S2 is a value within the range of from 2 to 10.
